# EUROPEAN PATENT SPECIFICATION

(11) **EP 2 661 234 B1**
(45) Date of publication and mention of the grant of the patent: **23.11.2016**
(21) Application number: 12700758.1
(22) Date of filing: 06.01.2012
(51) Int. Cl.: A61B 17/88

(54) **HYDRAULIC INJECTION SYSTEM FOR BONE CEMENT**
HYDRAULISCHES INJEKTIONSSYSTEM FÜR KNOCHENZEMENT
SYSTÈME D'INJECTION HYDRAULIQUE POUR CIMENT OSSEUX

(30) Priority: 06.01.2011 US 201161430435 P
(43) Date of publication of application: 13.11.2013
(73) Proprietor: Synthes GmbH, 4436 Oberdorf (CH)
(72) Inventor: ROTH, Christian, CH-4436 Oberdorf (CH); NEDUNKANAL, Suryo, CH-4436 Oberdorf (CH); SERTORE, Michele, CH-4436 Oberdorf (CH); WHEELER, Kurtis, CH-4436 Oberdorf (CH); CHAVATTE, Kris, CH-8700 Kuesnacht (CH); WEBER, Markus, CH-4436 Oberdorf (CH); OBERLI, Joel, CH-4436 Oberdorf (CH)
(74) Representative: Metzler, Volker
(86) International application number: PCT/US2012/020403
(87) International publication number: WO 2012/094549

(56) References cited:
- EP-A1- 1 570 873
- WO-A1-2010/089621
- WO-A2-2005/030034
- DE-A1- 4 022 986
- US-A1- 2004 260 303

## Description

### FIELD OF THE DISCLOSURE

The present disclosure generally relates to a hydraulic injection system, and in particular relates to a hydraulic injection system that is configured to deliver bone cement to a target anatomical location, such as a location remote from a radiation field.

### BACKGROUND

Cement augmentation procedures are a rapid growing field within orthopedic surgery and interventional radiology, including fracture stabilization in spinal indications, palliative tumor treatment and screw reinforcement. While being a valuable tool to surgeons, cement augmentation procedures have certain limitations. First, most cement injections are performed in combination with imaging, such as fluoroscopy or CT to monitor the expansion of the cement in vivo. Thus, surgeons frequently perform these procedures within the field of radiation and continued repeated exposure to potentially harmful effects of radiation. Second, certain bone cement augmentation procedures include multiple injection areas, for example, bi-pedicular Vertebroplasty and Kyphoplasty. Surgeons often spend costly time switching between the various injection areas as well as switching between more than one injection devices. Finally, cement viscosity issues can present problems in reliably delivering bone cement to a desired target area. If the bone cement viscosity is too high for the injection system being utilized, the cement may not be properly injected or cannot be injected at all. Alternatively, if low viscosity cements are utilized, to the cement may flow from the target area after injection and cause leakage, tissue damage and other problems.

WO 2010/089621 A1 discloses a dispensing unit for mixers of biphasic compound cements, including a piston that may slide in a mixing chamber. The piston can be driven by means of a hydraulic drive unit that includes a hydraulic fluid that can be pumped by means of a plunger from a reservoir through a fluid chamber and a conduit towards the piston to drive the piston and eject the cement from the mixing chamber.

EP 1 570 873 A1 discloses a manual pump mechanism and hydraulic delivery system, wherein a hydraulic pump is connected via a connecting tube with a syringe-like cartridge. By actuating a handle of the pump, hydraulic fluid is pumped towards the cartridge to act on a plunger and eject bone cement. A similar pump is described in DE 40 22 986 A1.

US 2004/0260303 A1 also discloses a system for delivering bone cement into a vertebra that includes a syringe and a cannula. The syringe includes a slidable piston for advancing bone cement. A fluid-driven system may be used to advance the piston within the syringe.

### SUMMARY

In accordance with the present disclosure, a hydraulic injection system is configured to deliver bone cement to a target anatomical location. The hydraulic injection system includes a cement cartridge assembly, a hydraulic injector, and at least one conduit. The cement cartridge assembly includes a plunger and a cartridge body that defines an interior cavity that slidably supports the plunger such that the plunger defines a barrier surface that is movable in the interior cavity from a first position to a second position so as to eject fluid out the interior cavity. The hydraulic injector includes a housing that defines a fluid chamber, a fluid reservoir in fluid communication with the fluid chamber, a piston that has a first piston end that is at least partially disposed within the fluid chamber, a suction check valve that is disposed between the fluid reservoir and the fluid chamber. The suction check valve allows fluid to travel along a direction from the fluid reservoir toward the fluid chamber, and prevents fluid from traveling from the fluid chamber to the fluid reservoir. The hydraulic injection further includes at least one outlet port that is in fluid tight communication with the fluid chamber. The conduit is configured to fluidly interconnect the at least one outlet port of the hydraulic injector and the cement cartridge assembly so as to place the fluid chamber and the interior cavity of the cartridge body in fluid communication with each other. The cement cartridge assembly includes at least two cement cartridges.

The hydraulic injector includes a housing, a fluid reservoir supported by the housing, and a piston. The piston defines a first piston end that is at least partially disposed within a fluid chamber defined by the housing. The hydraulic injector further includes a check valve disposed between the fluid reservoir and the fluid chamber, such that the fluid reservoir and the fluid chamber are in fluid tight communication with one another. The hydraulic injector further defines at least one outlet port that is in fluid tight communication with the fluid chamber. The injector and cement cartridge assembly are connected via at least one length of tubing connectively attached to and in fluid tight communication with the at least one outlet port of the hydraulic injector and the cartridge cap of the cement cartridge assembly. According to one embodiment, the tubing length is sufficient to remove an operator/user from a field of radiation. For instance, the tubing can be at least 50cm long.

According to the invention the check valve is a suction check, such as a low crack pressure check valve. According to one embodiment the low crack pressure check valve can have a crack pressure substantially equal to or greater than 0 kPa and less than 100 kPa. For instance, the crack pressure can be less than 50 kPa, such as less than 20 kPa, and in one embodiment less than 10 kPa.

According to one embodiment, the hydraulic injector further includes an outlet control valve assembly having an outlet control valve body and an outlet control switch disposed between the fluid chamber and the at least one outlet port. The outlet control switch includes a plug portion seated in the outlet control valve, and the plug portion includes at least two switch channels configured to selectively align with a valve inlet channel and at least one valve outlet channel in the outlet control valve body such that a fluid flow between the fluid chamber and the at least one outlet port can be controlled by a selective alignment of the outlet control switch. According to a further embodiment of the disclosure, the at least one valve outlet channel and the at least one outlet port includes two outlet valve channels and two outlet ports respectively.

The system can generate a pressure sufficient to inject a bone cement. According to an embodiment, the system can generate a pressure to inject a high viscosity bone cement. For example, in one embodiment, the system can generate a pressure in a range of about 0 to at least 20 bars; in another the system can generate a pressure in a range of about 0 to at least 50 bars; in yet another the system can generate a pressure in a range of about 0 to at least 80 bars; in still another the system can generate a pressure in a range of about 0 to at least 100 bars; and in a further the system can generate a pressure in a range of about 0 to at least 200 bars. Alternatively, the system can generate pressure sufficient to inject a low viscosity bone cement.

According to one embodiment the second distal portion of the cement cartridge body is configured to house a bone cement. In a further embodiment, the second distal portion of the cement cartridge body is configured to house a high viscosity bone cement.

[0001] According to one embodiment, the injector can further include a discharge check valve that is disposed between the fluid chamber and the at least one outlet port along a fluid flow path such that the fluid chamber and the at least one outlet port are in fluid tight communication with one another. In another embodiment, the discharge check valve is disposed between the fluid chamber and the outlet control valve assembly along a fluid flow path such that
the fluid chamber and the outlet control valve assembly are in fluid tight communication with one another.

According to one embodiment, the system can further include one or more cannulated needles that are adapted to access a target anatomical site or sites for the delivery of bone cement from the cement cartridge assembly.

According to one embodiment, the system can further include a hydraulic fluid conduit at least partially disposed within the cartridge cap. The hydraulic fluid conduit is configured to fluidly interconnect the at least one conduit and the interior cavity.

According to one embodiment, the piston is configured to move with respect to the housing between a first position and a second position such that movement of the piston from the first position to the second position causes hydraulic fluid in the fluid chamber to be ejected out of the housing through the at least one outlet port, and movement of the piston from the second position to the first position causes hydraulic fluid in the fluid reservoir to travel to the fluid chamber through the suction check valve.

According to one embodiment, the cartridge cap defines an inner channel and a transverse channel that is in fluid communication with the inner channel. The transverse channel leads to the outside environment. The cement cartridge assembly includes a venting valve that is configured to move between an open position in which the transverse channel is blocked, thereby precluding gases in the inner channel from exiting to the outside environment, and a closed position in which the transverse channel is in fluid communication with the outside environment, thereby allowing gases in the inner channel to escape to the outside environment.

A method is disclosed for injecting bone cement to a target anatomical location including:
introducing cement into an interior cavity of a cement cartridge;
connecting the cement cartridge to a cannulated needle that is configured to access a target anatomical location so as to place the interior cavity of the cement cartridge in fluid communication with the needle;
connecting the cement cartridge to a hydraulic injector via a conduit such that the injector can deliver a pressurized fluid to the cartridge through at least one of first and second outlet port of the injector;
actuating an outlet control valve assembly of the hydraulic injector so as to direct pressurized fluid can be selectively directed from the injector, through the outlet control valve assembly, and into at least a select one of the first and second outlet ports;
actuating a piston of the injector to force fluid from the hydraulic injector to the cement cartridge, so as to hydraulically force bone cement from the cement cartridge through the cannulated needle to the target anatomical location.

The method can further include selectively moving a venting valve of the cement cartridge from a closed position to an open position to remove gases from the cement cartridge before actuating the piston.

A further method is disclosed for injecting bone cement to a target anatomical location including:
introducing a bone cement into an interior cavity of a cement cartridge;
   connecting the cement cartridge to a cannulated needle that accesses a target anatomical location;
   connecting a first end of the cement cartridge to a hydraulic injector that has a fluid reservoir, such that the interior cavity of the cement cartridge is in fluid communication with the fluid reservoir via a conduit such.that the injector can deliver a pressurized fluid from the fluid reservoir to the interior cavity through the conduit;
   connecting a second end of the cement cartridge to a cannulated needle that is configured to access a target anatomical location, so as to place the interior cavity of the cement cartridge in fluid communication with the needle;
   actuating the injector to force fluid from the fluid reservoir to the interior cavity, wherein the injector includes a suction check valve disposed between the piston and the fluid reservoir that prevents the pressurized fluid from traveling into the fluid reservoir; and
   causing the fluid from the fluid reservoir to act against the cement cartridge so as to hydraulically force bone cement from the cartridge through the cannulated needle to the target anatomical location.

The method can further include repeating the step of actuating the piston. The method can further include selectively moving a venting valve of the cement cartridge from a closed position to an open position to remove gases from the cement cartridge before actuating the piston.

### BRIEF DESCRIPTION OF THE DRAWINGS

The foregoing summary, as well as the following detailed description of preferred embodiments of the application, will be better understood when read in conjunction with the appended drawings. For the purposes of illustrating the hydraulic injection systems of the present application, there is shown in the drawings preferred embodiments. It should be understood, however, that the application is not limited to the precise arrangements and instrumentalities shown. In the drawings:
Fig. 1 is perspective view of a hydraulic injection system for injecting bone cement according to one embodiment of the invention including a hydraulic injector, a cement cartridge assembly, and conduits connected between the hydraulic injector and the cement cartridge assembly;
Fig. 2 is a side elevation view of a cement cartridge of the cement cartridge assembly illustrated in Fig. 1, shown attached to the conduit;
Fig. 3 is a sectional side elevation view of the cement cartridge illustrated in Fig. 2, taken along line 3-3;
Fig. 4 is a perspective view of a cartridge body of the cement cartridge illustrated in Fig. 2;
Fig. 5 is a cross-sectional side view of the cartridge body illustrated in Fig. 4, taken along section line 5-5;
Fig. 6 is a perspective view of a cartridge cap of the cement cartridge shown in Fig. 2 in accordance with an embodiment of the present disclosure;
Fig. 7 is a side elevation view of the cartridge cap illustrated in Fig. 6;
Fig. 8 is a cross-sectional side elevation view of the cartridge cap illustrated in Fig. 6, taken along section line 8-8 of Fig. 7;
Fig. 9 is a cross-sectional top view of the cartridge cap illustrated in Fig. 6, taken along section line 9-9 of Fig. 8;
Fig. 10 is a perspective view of a venting valve of the cement cartridge shown in Fig. 2;
Fig. 11 is a bottom view of the venting valve illustrated in Fig. 10;
Fig. 12A is a side elevation view of a cement cartridge attached to the tubing according to another embodiment;
Fig. 12B is a sectional side elevation view of the cement cartridge illustrated in Fig. 12A;
Fig. 13A is perspective view of a connector of the cement cartridge shown in Fig. 12A;
Fig. 13B is an end elevation view of the connector shown Fig. 13A;
Fig. 13C is a cross-sectional side view of the connector shown in Fig. 13A, taken along section line 13C-13C of Fig. 13B;
Fig. 14A is a sectional side elevation view of a cement cartridge according to an alternative embodiment;
Fig. 14B is a sectional elevation view of a cement cartridge according to an alternative embodiment;
Fig. 15A is a perspective view of a valve of the cement cartridge shown in Fig. 14A;
Fig. 15B is a front elevation view of the valve shown in Fig. 15A;
Fig. 15C is a cross-sectional side view of the valve shown in Fig. 15A, taken along section line 15C-15C of Fig. 15B;
Fig. 16 is a sectional side elevation view of a cement cartridge according to an alternative embodiment;
Fig. 17 is perspective view of the cartridge illustrated in to Fig. 2 shown attached to the conduit at a proximal end, and attached to a cannulated needle at an opposed distal end;
Fig. 18 is a sectional side elevation view of the cartridge illustrated in Fig. 17;
Fig. 19 is a perspective view of a plunger of the cartridge illustrated in Fig. 2;
Fig. 20 is a cross-sectional side view of the hydraulic injector of Fig. 1;
Fig. 21A is a perspective view of an outer housing of the hydraulic injection system illustrated in Fig. 1;
Fig. 21B is a top elevation view of the outer housing of Fig. 21A;
Fig. 21C is a side elevation view of the outer housing of Fig. 21 A;
Fig. 21D is a cross-sectional side view of the outer housing of Fig. 21A, taken along section line 21D-21D of Fig. 21B;
Fig. 21E is a cross-sectional top view of an end portion of the outer housing of Fig. 21A, taken along section line 21E-21E of Fig. 21C;
Fig. 22 is a perspective view of a mechanical stop of the hydraulic injection system illustrated in Fig. 1;
Fig. 23 is a perspective view of a piston of the hydraulic injection system illustrated in Fig. 1;
Fig. 24 is a perspective view of a piston for the hydraulic injection system illustrated in Fig. 1 in accordance with another embodiment of the present disclosure;
Figs. 25A and 25B are perspective views of a plug portion of the hydraulic injection system illustrated in Fig. 1;
Fig. 25C is a side elevation view of the plug portion illustrated in Figs. 25A and 25B;
Fig. 25D is an end elevation view of the plug portion illustrated in Figs. 25A and 25B;
Fig. 25E is a cross-sectional side view of the plug portion illustrated in Figs. 25A and 25B, taken along section line 25E-25E of Fig. 25D;
Fig. 25F is a cross-sectional end view of the plug portion illustrated in Figs. 25A and 25B, taken along section line 25F-25F of Fig. 25E.
Fig. 26A is a cross-sectional end view of the of the control valve assembly illustrated in Fig. 20, taken generally along line 26-26 of Fig. 1, shown in an open position that provides for fluid flow from an inlet channel to both valve outlet channels;
Fig. 26B is a cross-sectional end view of the control valve assembly illustrated in Fig. 26A, shown in a closed position preventing fluid flow to either valve outlet channels;
Fig. 26C is a cross-sectional end view of the control valve assembly illustrated in Fig. 26A, shown in a first selectively open position that provides for fluid flow from an inlet channel to a select one of the valve outlet channels;
Fig. 26D is a cross-sectional end view of the control valve assembly illustrated in Fig. 26A, shown in a second selectively open position that provides for fluid flow from the inlet channel to the other valve outlet channel;
Fig. 27 is a perspective view of the hydraulic injection system according to another embodiment of the disclosure but not according to the invention including an alternatively embodied syringe-based hydraulic injector, tubing and a cement cartridge assembly;
Fig. 28 is a perspective view of a hydraulic injection system similar to Fig. 1, but including a hydraulic injector constructed in accordance with another embodiment not according to the invention,
Fig. 29 is a perspective view of a hydraulic injection system illustrated in Fig. 28, shown with a cannulated needle removed;
Fig. 30 is a sectional side elevation view of the hydraulic injection system illustrated in Fig. 28;
Fig. 31 is an enlarged sectional side elevation view of a portion of the hydraulic injection system illustrated in Fig. 30, indicated at Region A;
Fig. 32A is a perspective view of an outer housing of the hydraulic injection system illustrated in Fig. 28;
Fig. 32B is a cross-sectional side view of the outer housing of Fig. 32A, taken along section line 32B-32B of Fig. 32A;
Fig. 33 is a perspective view of a hydraulic injector similar to Fig. 28, but including more than one outlet port in accordance with another embodiment.
Fig. 34 is a side elevation view of the hydraulic injection system illustrated in Fig. 1, including a cannulated needle coupled to the cartridge assembly;
Fig. 35 is a perspective view of the hydraulic injector of Fig. 34 with a portion of the housing removed to show certain components within the housing including a fluid reservoir, an outlet control valve assembly, and a pressure chamber connected between the fluid reservoir and the outlet control valve;
Fig. 36 is a sectional side elevation view of the hydraulic injector of Fig. 34;
Fig. 37 is a schematic perspective view of the outlet control valve assembly illustrated in Fig. 35 including bifurcated valve outlet channels;
Fig. 38 is a sectional end elevation view of the control valve assembly illustrated in Fig. 11, taken generally along line 38-38, shown in an open position that provides for fluid flow from an inlet channel to both valve outlet channels;
Fig. 39 is a sectional end elevation view of the control valve assembly illustrated in Fig. 38; shown in a closed position preventing fluid flow to either valve outlet channels;
Fig. 40 is a sectional end elevation view of the control valve assembly illustrated in Fig. 38, shown in a first selectively open position that provides for fluid flow from an inlet channel to a select one of the valve outlet channels;
Fig. 41 is a sectional end elevation view of the control valve assembly illustrated in Fig. 38, shown in a second selectively open position that provides for fluid flow from the inlet channel to the other valve outlet channel;
Fig. 42 is a perspective view of an outlet control valve assembly in accordance with another embodiment, including an outlet control valve and an outlet control switch; and
Fig. 43A - 43D are schematic side elevation views of various examples of fluid reservoirs configured to be included in a hydraulic injector of the type illustrated in Fig. 34.

### DETAILED DESCRIPTION OF THE DRAWINGS

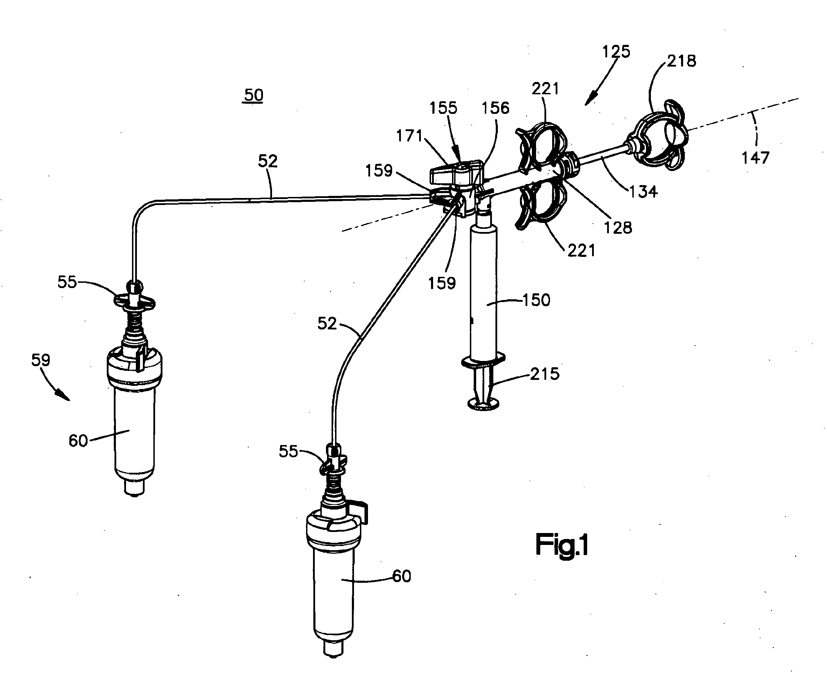

Referring to Fig. 1, a hydraulic injection system 50 configured to inject bone cement to at least one target anatomical location includes a hydraulic injector 125, a cement cartridge assembly 59 that includes at least one cement cartridge 60 (in embodiments according to the invention at least two, as shown), and one or more conduits 52, such as tubing, connected between the hydraulic injector 125 and the cartridge assembly 59 so as to place the hydraulic injector 125 in fluid communication with the at least one cement cartridges 60. The hydraulic injection system 50 is able to generate a sufficient hydraulic force to reliably inject bone cement, including high viscosity bone cement, to the anatomical location. For instance, in one embodiment, the hydraulic injection system 50 is configured to reliably generate a hydraulic pressure substantially equal to or greater than approximately 0 bars and less than approximately 200 bars, for instance less than approximately 100 bars. In accordance with one embodiment, the hydraulic injection system 50 is configured to reliably generate less than approximately 50 bars, such as less than approximately 20 bars.

The hydraulic injector 125 is configured to selectively direct a pressurized fluid through one or more outlet ports 159. According to one embodiment, the hydraulic injector 125 includes an outlet control assembly that is configured to selectively supply and regulate pressurized hydraulic fluid. For instance, the hydraulic injector 125 can include an outlet control switch 171 that can be actuated to selectively direct pressurized fluid flow through a particular outlet port or ports 159 as desired. In the example shown, the injector 125 includes two outlet ports 159, and the user can selectively allow pressurized fluid flow through one, both, or none of the outlet ports 159, by adjusting the position of the outlet control switch 171. It should be appreciated that while the example figures disclose an injector including two outlet ports 159, more or less than two outlet port may be included as desired. For example injector 125 can include a single port for a dedicated fluid flow, or can include more than two outlet ports, for example three, four or more outlet ports as desired. The pressurized fluid that flows from the outlet port(s) 159 further flows through the conduit 52 to a cartridge 60 where it is able to exert a force sufficient to push or expel a quantity of bone cement from the cartridge 60.

The conduit 52 can be any length as desired, and can have a length that permits a user to safely operate the injector 125 at a distance outside of any radiation field generated by fluoroscopic devices (e.g., X-ray or CT imagery devices) used to assist the placement of the bone cement to a target anatomical location. In one embodiment, the conduit 52 is at least 50 cm long. The conduit 52 can have a length ranging between about 70 cm and about 100 cm. In an embodiment, the conduit 52 can be tubing. The conduit 52 can be made from a substantially flexible material. The conduit 52 can be of sufficient structural integrity to withstand the pressures generated by the system 50, for example, at a range having a lower end substantially equal to or greater than approximately 0 bar, and an upper end that can be less than approximately 200 bar, such as less than approximately 100 bar, for instance less than approximately 80 bar, less than approximately 50 bar in accordance with one embodiment, and further less than approximately 20 bar in accordance with one embodiment. Thus, the hydraulic injection system 50 is able to reliably and safely inject a quantity bone cement including low and high viscosity bone cement to a target anatomical location or locations at a distance sufficient to remain outside a fluoroscopic field of radiation. In one embodiment according to the disclosure the hydraulic injection system can be used to perform vertebroplasty procedures, and particularly bi-pedicular vertebroplasty procedures. In another embodiment, the hydraulic injection system can be used to inject bone cement to assist the stabilization of a primary fixation device, for example a screw, or a bone plate, by providing secondary fixation.

### The Cement Cartridge

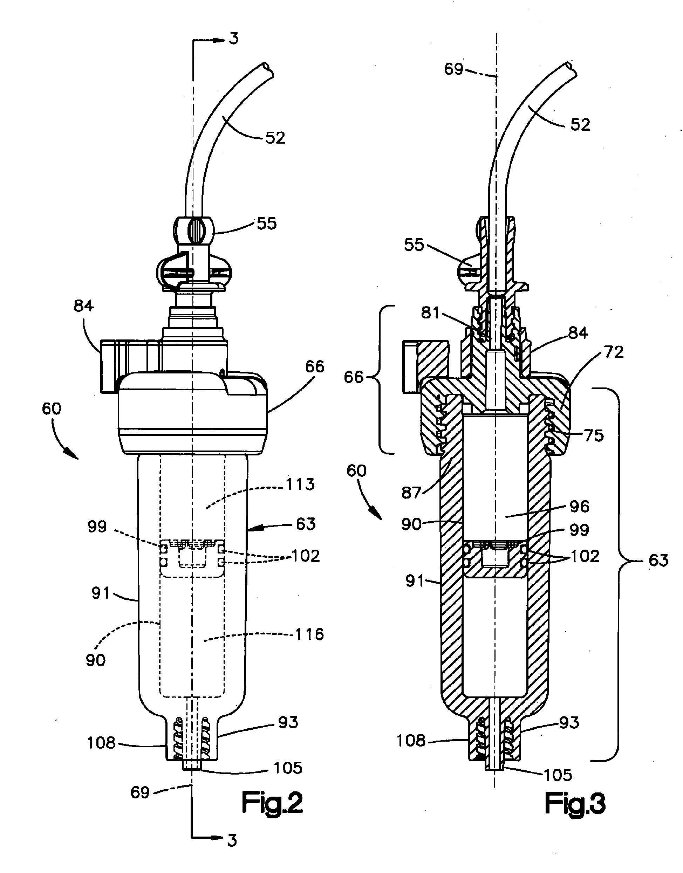

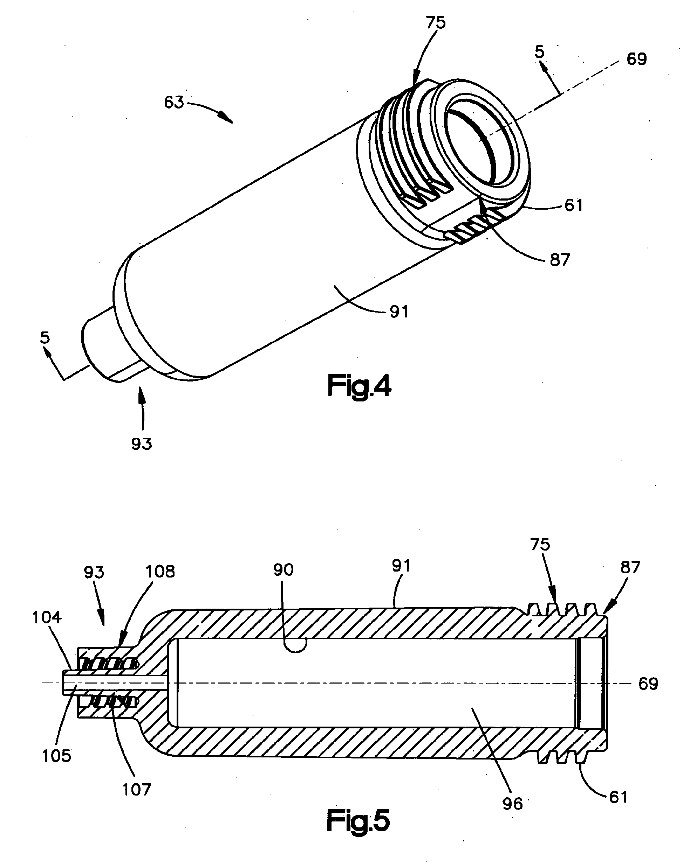

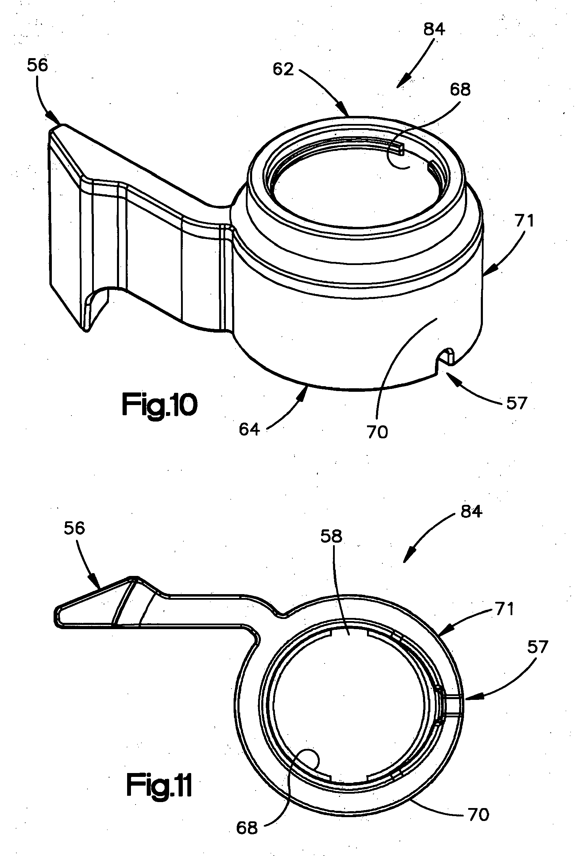

Referring to Figs. 2 - 10, each of the cartridges 60 includes a cartridge body 63 elongate along a central axis 69, and a cartridge cap 66 that is attached to a proximal end of the cartridge body 63 along the central axis 69. The cartridge body 63 can have any suitable shape. For instance, in the depicted embodiment, the cartridge body 63 has a substantially cylindrical shape. Regardless of its shape, the cartridge body 63 defines an outer surface 91, an inner surface 90, a first or proximal end 87, and a second or distal end 9. Both the outer surface 91 and the inner surface 90 extend along the central axis 69 from the proximal end 87 to a distal end 93 of the cartridge body 63. The cartridge body 63 includes a cap fastener 75 disposed at or near the proximal end 87. The cap fastener 75 is configured to couple the cartridge body 63 with the cartridge cap 66. In the depicted embodiment, the cap fastener 75 includes external threads 61 formed on the outer surface 91 of the cartridge body 63.

In an embodiment, the cartridge cap 66 is configured to fluidly couple the conduit 52 with the cartridge body 63, and includes a fastener 72 configured to connect with the cap fastener 75 located at a proximal end 87 of the cartridge body 63 such that cap 66 and body 63 are in fluid tight communication with one another. In the examples shown, the fastener 72 is coupled to the cap fastener 75 in a Luer lock fitting that is known in the art. In the depicted embodiment, the fastener 72 includes a body 21 that defines an outer surface 76 and an inner surface 77. The inner surface 77 defines an inner open cavity 79 that is sized and configured to fit over the cap fastener 75. The inner open cavity 79 terminates at an open end 73 that is sized and configured to receive at least a portion of the cartridge body 63, such as the cap fastener 75. The fastener 72 can further comprise an inner threads 74 formed on the inner surface 77. The inner threads 74 are disposed around the open cavity 79, and are configured to mate with the external threads 61 to connect the cartridge cap 66 to the cartridge body 63. The cap 66 further includes an inner channel 81 that extends axially along the central axis 69 when the cap 66 is connected to the cartridge body 63. The inner channel 81 extends through the cap 66 between the fastener 72 and a conduit fastener 78.

The cartridge cap 66 further includes the conduits fastener 78 that protrudes from the body 21 in a direction away from the open end 73. The conduit fastener 78 is configured to connect with a corresponding connector 55 of the conduit 52 such that conduit 52 and cap 66 are in fluid tight communication with one another. Thus, the conduit fastener 78 is configured to fluidly couple the conduit 52 with the cap 66. In the examples shown, the conduit fastener 78 is coupled to the connector 55 in a Luer lock fitting that is known in the art. In the depicted embodiment, the conduit fastener 78 includes a body 83 that defines an outer surface 85 and an inner surface 89. The conduit fastener 78 further includes a Luer taper 95 that is surrounded by the inner surface 89. A cavity 97, such as an annular space, is defined between the inner surface 89 and the Luer taper or tip 95. The cavity 97 is configured and sized to receive at least a portion of the connector 55. At least a portion of the inner channel 81 extends through the Luer taper 95. The conduit fastener 78 further defines threads 67 formed on the inner surface 89 such that the threads 678 surround the Luer taper 95. As can be appreciated, any number of suitable designs known in the art for connecting elements in fluid tight communication with one another are within the scope of the disclosure with respect to the fasteners of the cartridge assembly 59 described herein. In such a configuration as described, pressurized fluid is able to travel from the conduit 52 through the inner channel 81 of the cartridge cap 66 into the cartridge body 63 while the fasteners maintain fluid-tight communication and prevent a loss in pressure.

The cartridge cap 66 further defines a transverse channel 65 disposed in fluid communication with the inner channel 81. The transverse channel 65 terminates at an outlet 13 that leads to the outside environment. In the depicted embodiment, the transverse channel 65 extends from the outer surface 85 to the inner channel 81 in a direction that is substantially perpendicular to the central axis 69. As described in detail below, fluid flow through the transverse channel 65 can be controlled by a venting valve 84.

The cement cartridge 60 can additionally include at least one aeration venting valve 84 that permits the release of fluids and gases at a point in the fluid flow path between the tubing 52 and the cartridge body 63. During operation, the venting valve 84 allows for the removal of air (de-aerating) from the hydraulic injection system prior to injecting the bone cement in what is known in the art as "priming." For example, in the embodiment as shown in Figs. 2 - 3, the venting valve 84 is disposed on the cartridge cap 66 and allows for an opening in the inner cap channel 81 to the outside environment that can be manually opened or closed. For effective priming, the inlet of the hydraulic fluid has to be located lower than the outlet.

In the embodiment depicted in Figs. 2, 3, 12A, 14A, and 14B, the venting valve 84 includes a main body 71 that defines an outer surface 70 and an inner surface 68. The main body 71 may have a substantially cylindrical shape. It is envisioned, however, that the main body 71 may have any other suitable shape. The outer surface 70 can define a substantially circular or oval shape cross-section. However, the outer surface 70 can have a cross-section with any other suitable shape. The main body 71 further defines a first or proximal end 62 and a second or distal end 64. The inner surface 68 defines an opening 58 that extends from the proximal end 62 to the distal end 64. The opening 58 is configured and sized to receive at least a portion of the cartridge cap 66. For example, the opening 58 can be configured and sized to receive at least a portion of the conduit fastener 78 such that the venting valve 84 can rotate with respect to the cartridge cap 66. When the venting valve 84 is disposed around a portion of the cartridge cap 66, the opening 58 extends from the proximal end 62 to the distal end 64 along a direction that is substantially parallel the central axis 69. The main body 71 further defines a transverse channel 57 that extends from the outer surface 70 to the inner surface 68. In an embodiment, the transverse channel 57 can extend extends between the outer surface 70 and the inner surface 68 along a direction substantially perpendicular to the central axis 69 when the venting valve 84 is mounted on the cartridge cap 66. In the depicted embodiment, the transverse channel 57 is disposed at or near the distal end 64. It is envisioned, however, that the transverse channel 57 can be positioned at any other suitable location of the main body 71. In addition, when the venting valve 84 is mounted on the cartridge cap 66, the venting valve 84 can be rotated relative to the cartridge cap 66 so that the transverse channel 57 is in fluid communication with the transverse channel 65 of the cartridge cap 66 as described in detail below. To facilitate manual rotation, the venting valve 84 can include a lever 56 that protrudes outwardly from the outer surface 70 away from the opening 58 and inner surface 68. The lever 56 allows a user to rotate the venting valve 84 manually with respect to the cartridge cap 66.

The venting valve 84 is configured to move (.e.g., rotate relative cartridge cap 66) between an open position and a closed position. In the open position, the transverse channel 57 is substantially aligned and in fluid communication with the transverse channel 65 of the cartridge cap 66, causing the inner channel 81 to be in fluid communication with the outside environment. Hence, the venting valve 84 permits fluids passing through the inner channel 81 between the conduit 52 and the cartridge body 63 to be released to the outside environment when the transverse channel 57 is in fluid communication with the transverse channel 65 of the cartridge cap 66. In operation, a user can place the venting valve 84 in the open position to allow air removal (de-aerating) from the hydraulic injection system prior to injecting the bone cement in what is known in the art as "priming." When the venting valve 84 is in the closed position, the transverse channel 57 is not aligned with the transverse channel 65 of the cartridge cap 66. Therefore, the transverse channel 57 is not in fluid communication with the transverse channel 65 when the venting valve 84 is in the closed position. Accordingly, in the closed position, the venting valve 66 prevents, or least inhibits, the release of fluid passing through the inner channel 81 between the conduit 52 and the cartridge body 63 to the outside environment.

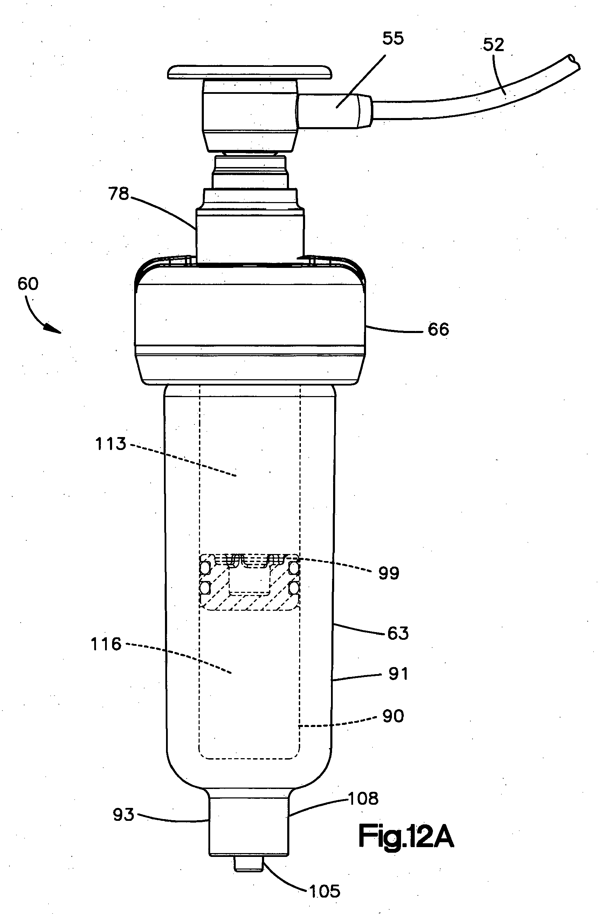

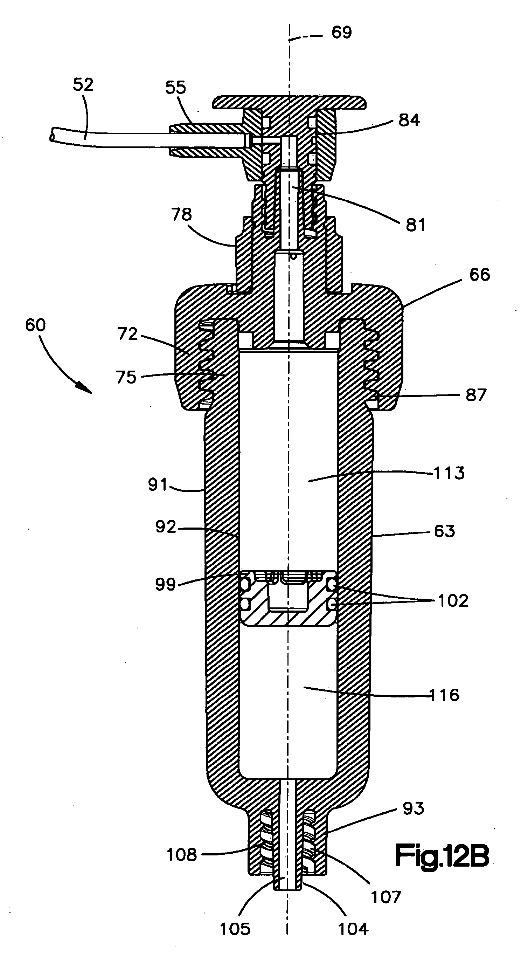

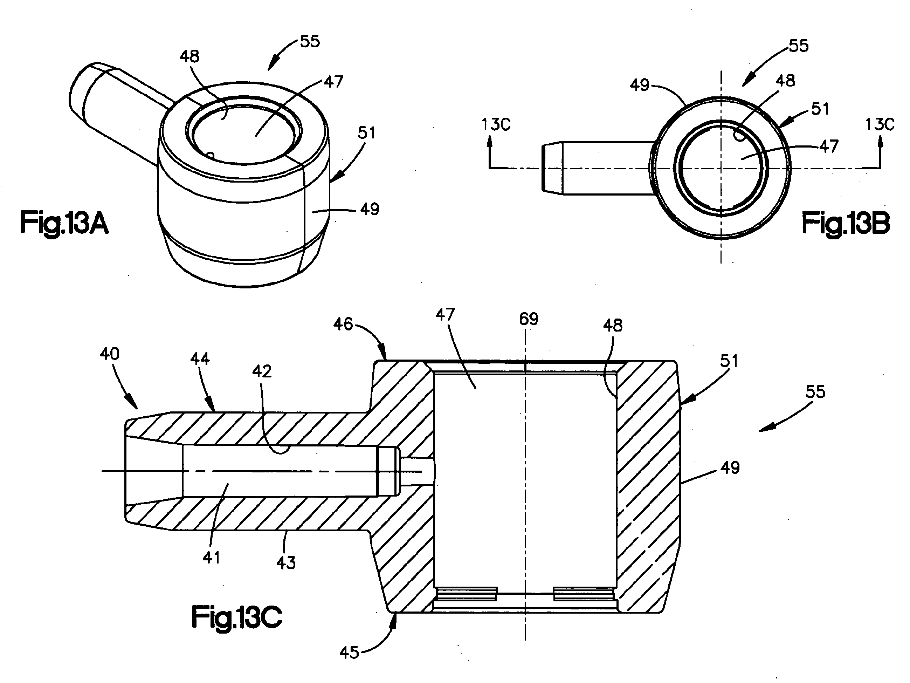

In the alternative embodiment as shown in Figs. 12A, 12B, and 13A-13C" the connector 55 includes a body portion 51 that defines an outer surface 49 and an inner surface 48. The inner surface 48 defines an opening 47 that is configured and sized to receive at least a portion of the venting valve 84 shown in Fig. 12B. The body portion 51 defines a first or proximal end 46 and a second or distal end 45. The opening 47 can extends from the proximal end 46 to the distal end 45 of the body portion 51. Moreover, the opening 47 can extend along the central axis 69 when it is mounted on the venting valve 84 shown in Fig. 12B. The connector 55 further includes a projecting member 44 that protrudes outwardly from the outer surface 49 of the body portion 51 in a direction away from the opening 47 and the inner surface 48. The projecting member 44 can extend in a direction that is substantially perpendicular to the central axis 69 when the connector 55 is mounted on the venting valve 84 shown in Fig. 12B. The projecting member 44 defines an outer surface 43 and an inner surface 42. The inner surface 42 defines an opening that extends in a direction substantially perpendicular to the central axis 69. The opening 41 can extend from the opening 47 defined by the body portion 51 toward a free end 40 of the projecting member 44. The opening 41 is configured and sized to receive a portion of the conduit 52 in order to connect the conduit 52 to the connector 55. A portion of the conduit 52 can be positioned in the opening 41 to establish a friction fit connection between the connector 55 and such portion of the conduit 52.

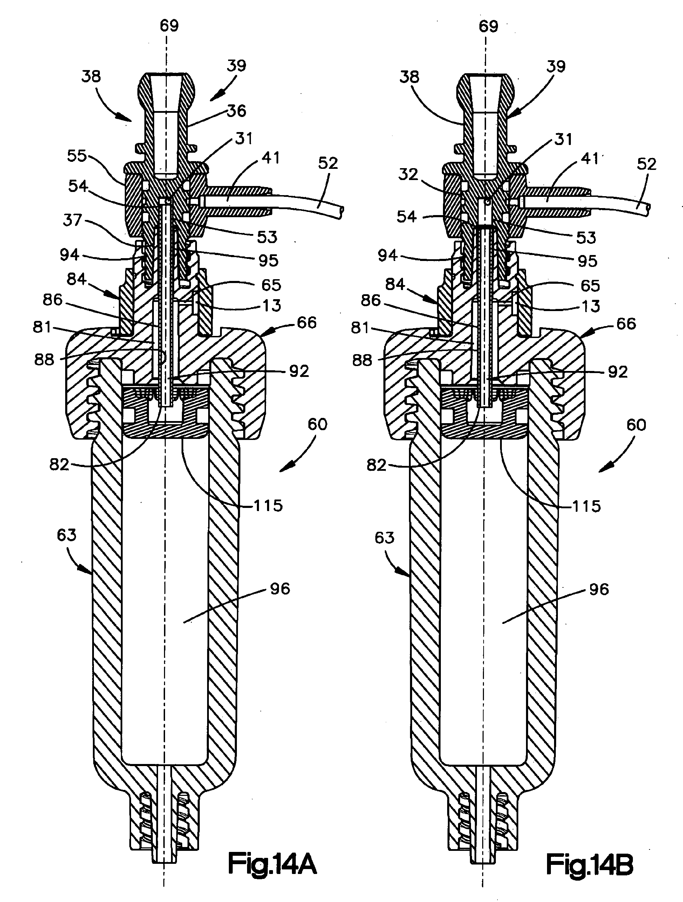

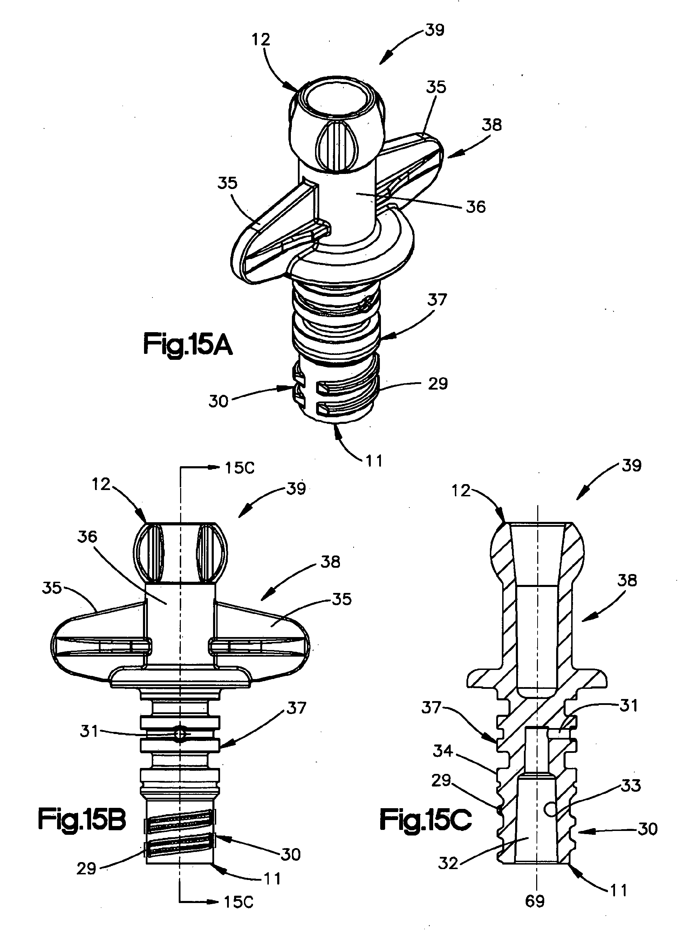

With reference to Figs. 14A-B and 15A-C, an embodiment of the cartridge assembly 60 includes a valve 39 configured to control the fluid entering the cartridge assembly 60 from the conduit 52. The valve 39 defines a first or proximal end 12 and a second or distal end 11. In the depicted embodiment, the valve 39 is a stopcock valve. However, the valve 39 can be any kind of valve capable of controlling the fluid flow between the conduit 52 and the cartridge assembly 60. In the depicted embodiment, the valve 39 includes knob 38 and a fastening section 37 that extends from the knob 38. The knob 39 is configured to facilitate rotation of the valve 39 by a user. Although the depicted valve 39 includes the knob 38, it is envisioned that valve 39 can include any kind of handle suitable for facilitating rotation of the valve 39 by a user. In the depicted embodiment, the knob 38 includes a body portion 36 and one or more wings 35 that extend outwardly from the body portion 36. The knob 38 can include a pair of wings 35 (see Figures 15A-C) that protrude radially outward from opposite sides of the body portion 36. The fastening section 37 extends downwardly from the body portion 36, and defines an outer surface 34 and an inner surface 33 (see Figures 15C). At least a portion of the fastening section 37 is configured and sized to be received in the opening 47 (Figs. 13A-13C) of the connector 55. The inner surface 33 defines an inner channel 32 that extends from a transverse channel 31 toward the distal end 11 of the valve 39. The inner channel 32 can extend along (or substantially parallel to) the central axis 69 when the valve 39 is connected to the cartridge assembly 60. The valve 39 further defines the transverse channel 39, which is disposed in fluid communication with the inner channel 32. The transverse channel 31 can extend from the inner channel 32 to the outer surface 34 in a direction substantially perpendicular to the central axis 69 when the valve 39 is coupled to the cartridge cap 66. The transverse channel 31 extends through the inner surface 33 and the outer surface 34 of the fastening section 37. In the depicted embodiment, the transverse channel 31 is disposed at the fastening section 37. However, the transverse channel 31 can be disposed at any other location along the valve 39 so long as it is in fluid communication with the inner channel 32.

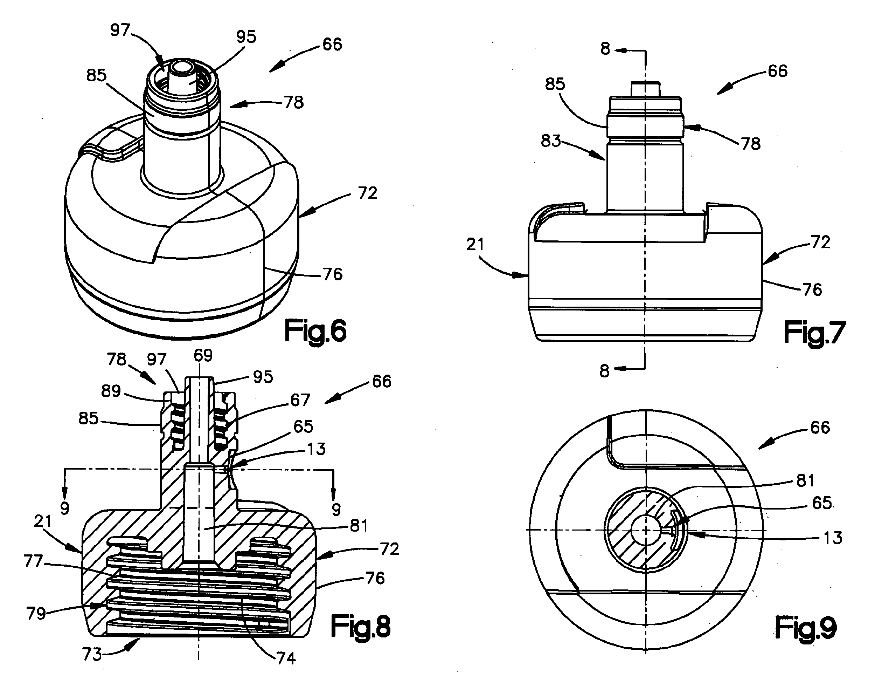

With continued reference to Figs. 14A-B and 15A-C, the fastening section 37 can include a threaded portion 30 that is configured and sized to be received in the cavity 97 (Figs. 6 and 8) of the cartridge cap 66. The threaded portion 30 includes external threads 29 formed on the outer surface 34 of the fastening section 37. The external threads 29 are configured to mate with the threads 67 (Fig. 8) of the cartridge cap 66 such that the cartridge cap 66 can be connected to the valve 39. When the valve 39 is connected to the cartridge cap 66, the inner channel 32 is fluid communication with the inner channel 81.

With continued reference to Figs. 14A-B and 15A-C, the cartridge assembly 60 can further include a hydraulic fluid conduit 53 that is elongate in a direction that is substantially parallel to the longitudinal axis 69. The hydraulic fluid conduit 53 can be connected to the valve 39 and/or the cartridge cap 66. For instance, the hydraulic fluid conduit 53 can be glued, pressed fitted, or welded to the cartridge cap 66 and/or the valve 39. Alternatively, the hydraulic fluid conduit 53 can be injection molded directly to the cartridge cap 66 and/or the valve 39. The hydraulic fluid conduit 53 is at least partially disposed within the cartridge cap 66. Further, the hydraulic fluid conduit 56 is configured to fluidly interconnect the conduit 52 and an interior cavity 96. The hydraulic fluid conduit 53 defines a first or proximal end 54, a second or distal end 82, an outer surface 86, and an inner surface 88. The inner surface 88 defines a channel 92 that extends from the proximal end 54 to the distal end 82 in a direction substantially parallel to the longitudinal axis 69. The hydraulic fluid conduit 53 is at least partially positioned in the inner channel 32 such that the channel 92 is in fluid communication with the opening 41 of the connector 55. In the embodiment depicted in Fig. 14A, the proximal end 54 of the hydraulic fluid conduit 53 can be disposed in substantial alignment with the opening 41 of the connector 55. Alternatively, the proximal end can be disposed in substantial alignment with an end 94 of the tip 95 as seen in Fig. 14B. At least a portion of the hydraulic fluid conduit 53 is disposed in the inner channel 81. Further, at least a portion of the hydraulic fluid conduit 53 extends into the interior cavity 96 of the cartridge body 63. For instance, at least the distal end 82 of the hydraulic fluid conduit 53 can be disposed in the interior cavity 96.

With continued reference to Figs. 14A-B and 15A-C In operation, the hydraulic fluid conduit 53 facilitates deaeration. As discussed above, the venting valve 84 can move between open and closed positions. When the venting valve 84 is the closed position, the outlet 13 of the transverse channel 65 is blocked and, therefore, gases, such as air, contained in the inner channel 81 cannot escape through the outlet 13. When the venting valve 84 is in the open position, gases in the inner channel 81 can escape to the outside environment through the outlet 13. If the hydraulic fluid conduit 53 is not at least partially disposed in the inner channel 81, the liquid, such as water, in the inner channel 81 can interfere with the gasses, thereby preventing, or at least inhibiting the gasses from exiting the cartridge assembly 60 via the outlet 13. In operation, the fluid channel 53 facilitates deaeration by directing the flow of liquid from entering through the transverse channel 31. Specifically, liquid, such as water, entering the cartridge assembly 60 through the transverse channel 31 flows into the channel 92 of the fluid conduit 32 and eventually reaches the inner cavity 96 of the cartridge body 63. When the venting valve 84 is in the open position, the gases, such as air, in the cavity 96 can flow through the portions of the inner channel 81 surrounding the outer surface 86 of the fluid conduit 32 and exit the cartridge assembly 60 through the outlet 13. In this case, the liquid passing through the channel 92 of the hydraulic fluid conduit 53 does not interfere with the gases and therefore allows the gasses the freely flow through the portion of the inner channel 81 surrounding the hydraulic fluid conduit 53. Thus, the venting valve 84 is configured to move between an open position in which the transverse channel 65 is blocked, thereby precluding gases in the inner channel 81 from exiting to the outside environment, and a closed position in which the transverse channel 95 is in fluid communication with the outside environment, thereby allowing gases in the inner channel 81 to escape to the outside environment.

With continued reference to Figs. 14A-B and 15A-C, the valve 39 is configured to move (e.g., rotate relative to the cartridge cap 66) between an open position and a closed position. In the open position, the transverse channel 31 is substantially aligned and in fluid communication with the opening 41 (Fig. 13C) of the connector 55 in order to establish fluid communication between the conduit 52 and the inner channel 32, thereby fluidly coupling the conduit 52 with the inner channel 81 of the cartridge assembly 60. Thus, in the open position, the valve 39 allows fluid to travel between the conduit 52 and the cartridge assembly 60. In the closed position, the valve 39 prevents, or at least inhibits, fluid from traveling between the conduit 52 and the cartridge assembly 60. In the depicted embodiment, the transverse channel 39 is not aligned with (i.e., not in fluid communication with) the opening 41 (Fig. 13C) of the connector 55 when the valve 39 is in the closed position, thereby precluding, or at least hindering, fluid from traveling between the conduit 52 and the cartridge assembly 60.

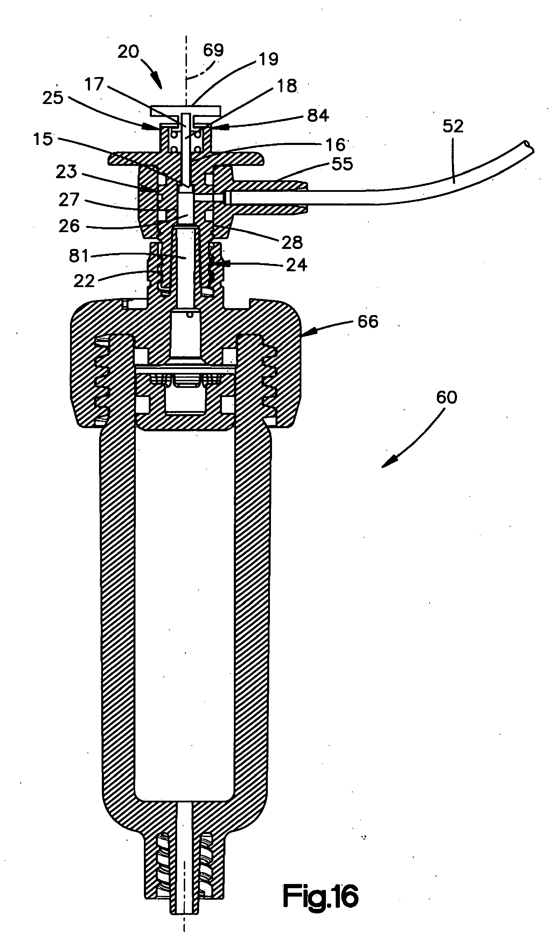

In still further embodiments, as shown in Fig. 16, the venting valve 84 is disposed at least partially within the connector 55 as shown in Figs. 13A-13C, similar to the embodiment shown in Figs. 12A; however in Fig. 16, the venting valve 84 is configured as a depressible button or disc that is configured to open and vent the system upon depression. In the depicted embodiment, the venting valve 84 includes an outer surface 28 and an inner surface 27. The inner surface 27 defines an inner channel 26 that extends along the central axis 69 when the venting valve 84 is connected to the cartridge 60. When the venting valve 84 is connected to the cartridge cap 66, the inner channel 26 is in fluid communication with the inner channel 81 of the cartridge 60. The inner channel 26 terminates at a vent 16 that leads to the outside environment. The venting valve 84 includes a proximal end portion 25, a distal fastening portion 24, and a central portion 23 that is disposed between the proximal end portion 25 and the distal fastening portion 24. The central portion 23 is configured and sized to be received within the opening 47 (Figs. 13A-13C) of the connector 55. The distal fastening portion 24 is configured to be received within the cavity 97 (Figs. 6 and 8) of the cartridge cap 66. In addition, the distal fastening portion 24 includes external threads 22 formed on the outer surface 28. The external threads 22 are configured to mate with the threads 67 of the cartridge cap 66 in order to couple the venting valve 84 with the cartridge cap 66. The proximal end portion 25 includes a depressible button or disc 19 configured to move along the central axis 69, and an elongated member 18, such as a rod, attached to the button 19. The elongated member 18 has a first or proximal end 17 that is connected to the button 19, and a second or distal enlarged end 15. The distal enlarged end 15 of the elongated member 18 is configured and sized to block the vent 16 when the venting valve 84 is in the closed position, thereby preventing fluid in the inner channel 27 and the inner channel 81 from being released to the outside environment. To move the venting valve 84 to the open position, the user can press the button downwardly (*i.e.,* toward the cartridge cap 66) to separate the distal enlarged end 15 from the vent 16. In the open position, the distal enlarged end 15 of the elongate member 18 does not block the vent 16 and therefore allows fluid in the inner channel 26 and inner channel 81 to exit to the outside environment.

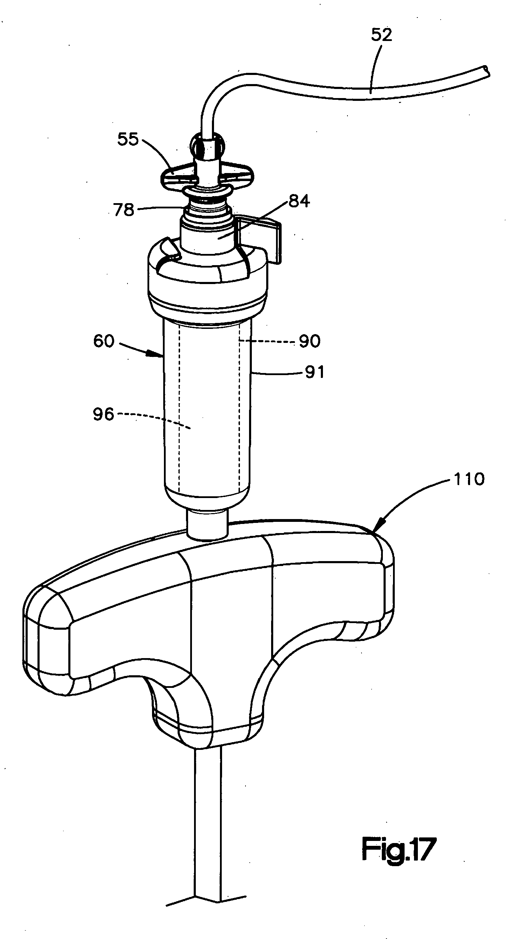

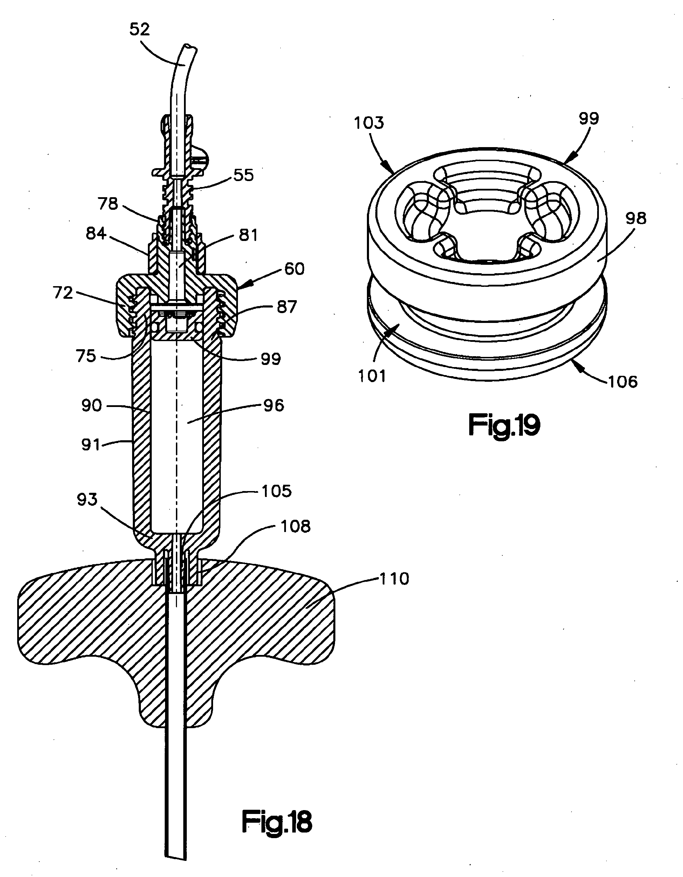

Referring further to Figs. 2 - 19, the cartridge body 63 can be shaped as desired, for example, as a common cylindrical syringe as shown. As discussed above, the outer surface 91 and the inner surface 90 of the cartridge body both extend along the central axis 69 from the proximal end 87 to the distal end 93 of the cartridge body 63. The inner surface 90 defines an interior cavity 96 having a fixed volume that is in fluid tight communication with the inner cap channel 81. A slidable plunger 99, such as a piston, is disposed within the cavity 96 and shaped to geometrically conform to the interior cavity 96. The plunger 99 can include at least one seal or sealing lip 102, for example an O-ring, circumferentially disposed along an outer periphery of the plunger 99 that engages the inner surface 90 in fluid tight communication. In the embodiment shown in Fig. 19, the plunger 99 defines a first or proximal end 103, a second or distal end 106, and an outer surface 98 that extends between the proximal end 103 and the distal end 106. The outer surface 98 defines at least one groove 101, such as an annular groove, that extends around the perimeter plunger 99. The groove 101 is disposed between the proximal end 103 and the distal end 106, and is configured and sized to tightly receive the seal or sealing lip 102. For instance, the groove 101 can be configured and sized to receive at least one O-ring. The plunger 99 is able to move axially in the cavity 96 and defines a variable barrier between a first fluid portion 113 of the cavity 96 located proximally to the plunger 99 and a second cement portion 116 of the cavity 96 located distal to the plunger 99. The second cement portion 116 can house a quantity of bone cement to be injected into a target anatomical location. Because the interior cavity 96 has a fixed volume, distal movement of the plunger 99 will cause an increase in volume of the first fluid portion 113 and an equivalent reduction in volume of the second cement portion 116. Thus, it can be appreciated that an inverse relationship exists between the volume of the first fluid portion 113 and the volume of the second fluid portion 116 that is dependent upon the axial movement of the plunger 99 within the cavity 96. The interior cavity 96 slidably supports the plunger 99 such that the plunger 99 defines a barrier surface 115 that is movable in the interior cavity 96 from a first position to a second position so as to eject fluid out the interior cavity 99.

The interior cavity 96 terminates at the distal end 93 as a discharge opening 105. In the depicted embodiment, the cartridge body 63 can include a Luer taper or tip 104 that defines the discharge opening 105. The cartridge 60 can further include a needle fastener 108 at the distal end 93 that is configured to connect with a cannulated needle 110, such as a trocar, for example a vertebroplasty needle, such that the cartridge body 63 and the cannulated needle 110 are in fluid-tight communication with one another. In the example shown, the needle fastener 108 is coupled to the proximal end of the cannulated needle 110 in a Luer lock fitting that is known in the art. In the depicted embodiment, the needle fastener 108 is disposed at or near the distal end 93 of the cartridge body 63, and includes inner threads 107 that are configured to mate with external threads of a needle or any other suitable injection apparatus. As can be appreciated, any number of suitable designs known in the art for connecting elements in fluid tight communication with one another are within the scope of the disclosure with respect to the fasteners of the cartridge assembly described herein.

During operation, pressurized fluid enters the cavity 96 proximal to the plunger 99 in the first fluid portion 113. As the pressurized fluid fills the first fluid portion 113, it exerts an axial force on the plunger 99 that biases the plunger 99 distally along the central axis 69, thereby causing an increase in the volume of the first fluid portion 113 and decreasing the volume of the second cement portion 116. As the pressurized fluid continues to fill and expand the first fluid portion 113 and force the plunger distally, bone cement housed within the second cement portion 116 can be ejected through the discharge tip 105 into the cannulated needle 110 and ultimately to the target anatomical location. Thus, it should be appreciated that the hydraulic injection system as disclosed herein can further include any type of cannulated needle or lumen suitable for the application of bone cement from the described cement cartridge to the target anatomical location.

### The Hydraulic Injector

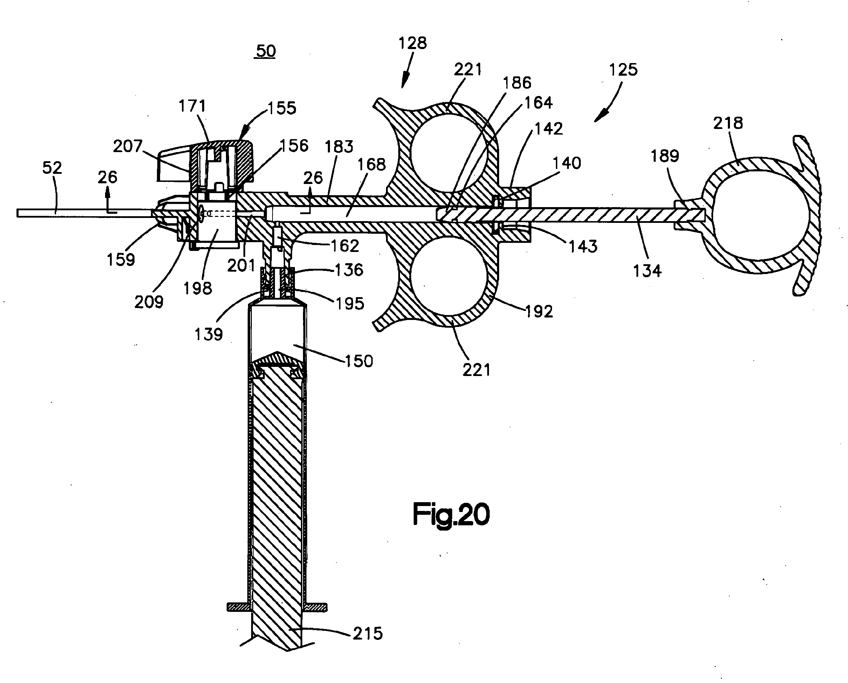

Referring now to Figs. 1 and 20, the hydraulic injection system 50 can include a hydraulic injector 125 constructed to deliver pressurized fluid to the cartridge assembly 59. In the embodiment as shown, the injector 125 includes an outer housing 128 shaped as a syringe that defines an interior fluid chamber or channel 168 disposed within the housing 128 along a central axis 147. The injector 125 further includes a fluid reservoir 150, a piston 134, and one or more outlet ports 159. In certain embodiments, the fluid reservoir 150 can be configured as a syringe adapted to house a fluid, and the fluid reservoir 150 can further include a reservoir plunger 215. The fluid reservoir 150 is configured to hold any suitable hydraulic fluid. Suitable hydraulic fluid include, but are not limited, to water and saline. Moreover, the hydraulic fluid can be a liquid or a gas. As illustrated in Figs. 1 and 20, the injector 125 further includes an outlet control valve assembly 155 that, in turn, includes a valve body 156 and an outlet control switch 171 connected to the valve body 156. Accordingly, the injector 125 can bifurcate the pressurized fluid flow through more than one outlet port 159 as described below. In an embodiment, the valve body 156 is part of the housing 128. For instance, the valve body 156 can be monolithically formed with the housing 128. The conduit 52 is configured to fluidly interconnect at least one outlet port 159 of the hydraulic injector 125 and the cement cartridge assembly 59 so as to place the fluid chamber 168 and the interior cavity 96 of the cartridge body 63 in fluid communication with each other.

The injector 125 defines a fluid flowpath from the reservoir 150, through the fluid chamber 168, further through the outlet control valve assembly 155, and finally through the outlet port(s) 159. The fluid chamber 168 is peripherally defined by an the outer housing 128, and the piston 134 has a first end 186 that is at least partially disposed within the fluid chamber 168. The first piston end 186 includes at least one piston seal 192, for example an O-ring, circumferentially disposed about an outer periphery of the first piston end 186 that engages the shaft wall 183 in fluid tight communication. At least a portion of the piston 134 can extend beyond the fluid chamber 168 and includes a second piston end 189 that can include a piston grip 218 configured to be manually engaged by a user so as to actuate the piston 134. In accordance with the illustrated embodiment, the piston grip 218 is configured as a finger ring. As it can be appreciated, the piston grip 218 can be any of a variety of shapes or configuration as desired such that it can assist a user in manual actuation of the piston 134.

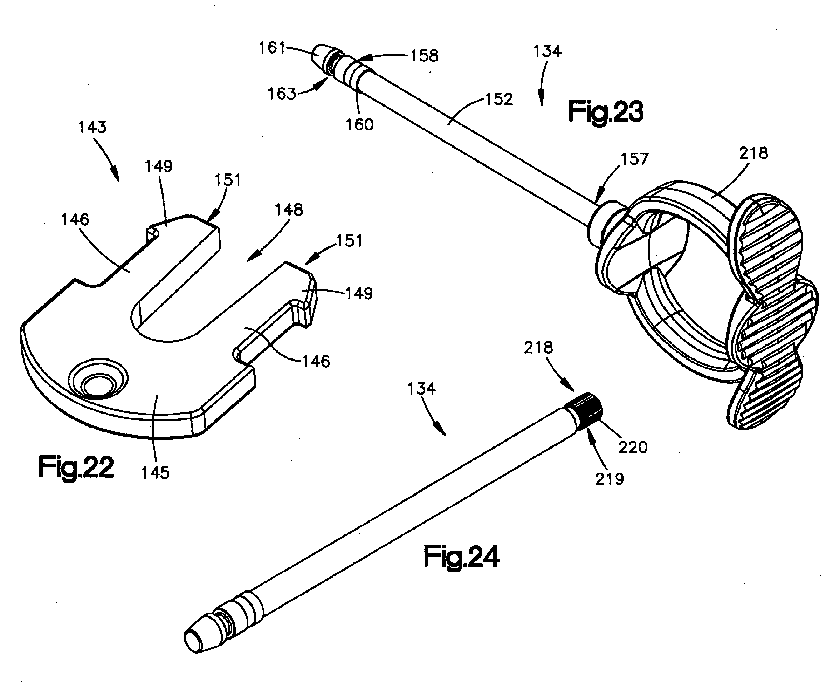

In an embodiment of the piston 134 shown in Fig. 23, the piston grip 218 is made from plastic, and is attached to a shaft 152 of the piston 134. The plastic piston grip 218 can be molded onto the shaft 152, which can be made from metal. The shaft 152 includes a proximal or first end portion 157 and a distal or second end portion 158. The piston grip 218 is attached, such as molded onto, the proximal end portion 157 of the shaft 152. The distal end portion 158 of the shaft 152 includes a shoulder 160 and tip 161. The tip 161 can have a substantially frusto-conical shape. The distal end portion 158 can further define a groove 163 that extends about the perimeter of the shaft 152. The groove 163 is configured and sized to receive a seal 164 (Fig. 20), such as an O-ring. The seal 164 is configured to establish a fluid tight connection between the piston 134 and the housing 128.

In another embodiment of the piston 134, the piston 134 can be partly or entirely made from metal and does not include a piston grip 218 in the shape of a ring. Rather, the piston grip 218 of this embodiment includes a substantially solid body 219 that has a knurled outer surface 220. The solid body 219 can have a substantially cylindrical shape.

The housing 128 further includes at least one brace 221 that can provide additional control and/or support for the user in operating the injector 125. In the example as shown, there are two braces 221 configured as finger rings disposed on opposing sides of the housing 128 separated by axis 147. As it can be appreciated, the braces 221 can be any of a variety of shapes or configuration as desired, including more or less than two, such that it can assist a user in control and support of the injector 125.

The injector 125 further includes a suction check valve 162 disposed between the fluid reservoir 150 and the fluid chamber 168 along a fluid flowpath and allows the fluid to flow unidirectionally from a reservoir opening 195 in the fluid reservoir 150 into the fluid chamber 168, and operates in the manner as described above. The reservoir 150 can be connected to the housing 128 by a fluid tight connection between the reservoir opening 195 and the suction check valve 162, for example via Luer lock connection, and can be directly connected as shown in the example figures, or can alternatively be indirectly connected via tubing as has been previously described. Additionally, as has been previously described the reservoir 150 can be formed as an integral part of the housing 128. The suction check valve 162 is disposed between the fluid reservoir 150 and the fluid chamber 168. In operation, the suction check valve 162 allows fluid to travel along a direction from the fluid reservoir 150 toward the fluid chamber 168, and prevents fluid from traveling from the fluid chamber 168 to the fluid reservoir 150.

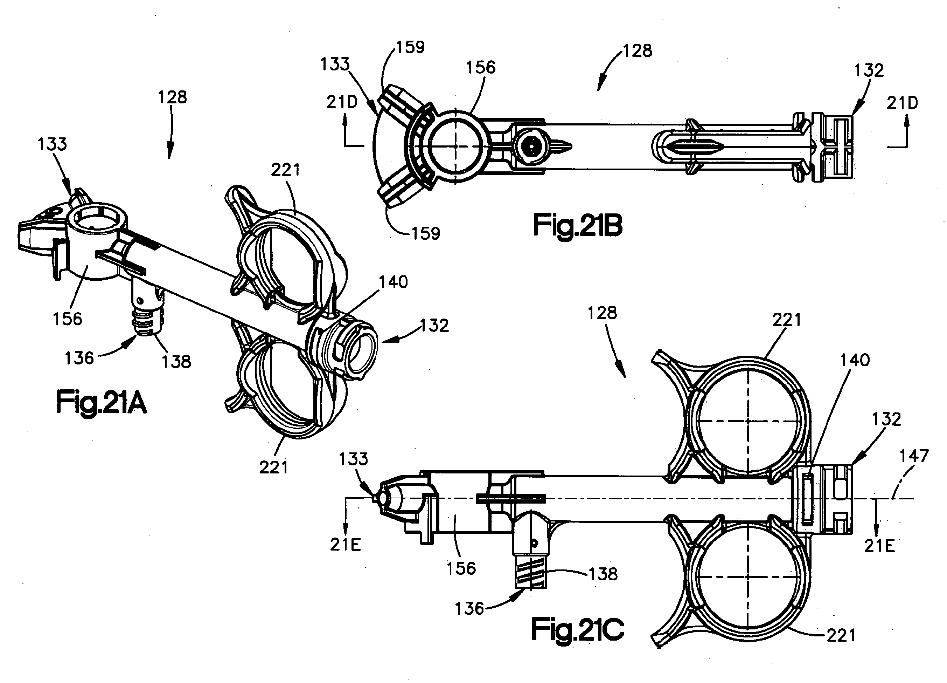

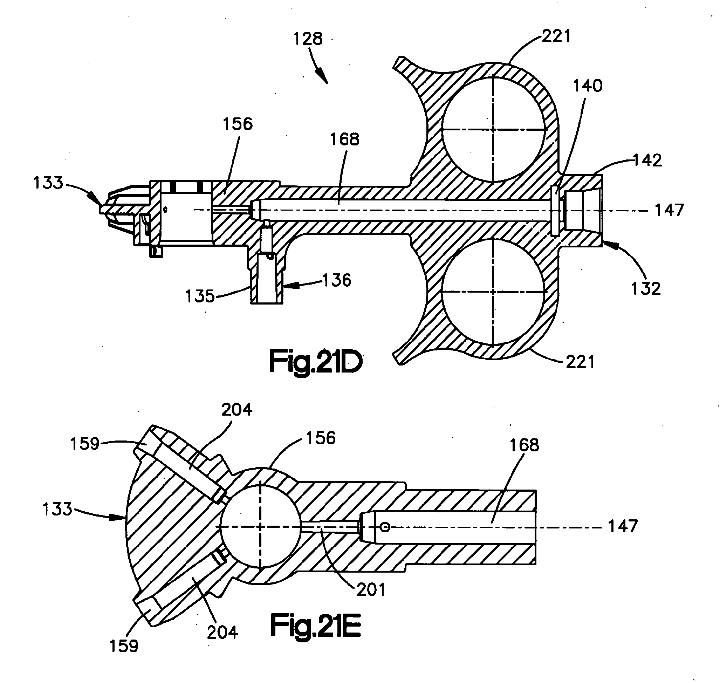

In an embodiment of the housing 128 depicted in Figs. 21A-21E, the housing 128 defines a first or proximal end 132 and a second or distal end 133. The fluid chamber 168 extends from the proximal end 132 and terminates at the valve body 156, so that the fluid chamber 168 is in fluid communication with the outlet control valve assembly 155. As discussed above, the valve body 156 of the outlet control valve assembly 155 can be part of the housing 128. The housing 128 further defines a transverse channel 135 that can be substantially perpendicular to the central axis 147. The transverse channel 135 is configured and sized to receive the suction check valve 162, and is configured to establish fluid communication between the fluid reservoir 150 and fluid chamber 168. The housing 128 further comprises a reservoir fastener 136 disposed about at least a portion of the transverse channel 135. The reservoir fastener 136 can be, for example, a Luer lock connection, or any other fastener capable of fluidly coupling the fluid reservoir 150 with the fluid chamber 168. For instance, the reservoir fastener 136 can include external threads 138 configured to mate with inner threads 139 (Fig. 20) of the fluid reservoir 150.

The housing 128 can further define an outer surface 142 and a slot 140 at or near its proximal end 132. The slot or opening 140 extends from the piston receiving channel 168 toward the outer surface 142. In the depicted embodiment, the slot 140 extends in a direction substantially perpendicular to the central axis 147. The slot 140 is configured and sized to receive a mechanical stop 143. The mechanical stop 143 is configured to prevent, or at least inhibit, the piston 134 from completely exiting the fluid chamber 168 when the piston 134 is moved in a proximal direction (i.e., away from the outlet control valve assembly 155). In the embodiment depicted in Fig. 22, the mechanical stop 143 includes a body portion 145 and a pair of legs 146 that extend from the body portion 145. The body portion 145 and the legs 146 collectively define a notch or opening 48 that is configured and sized to receive at least a portion of the piston 134. The legs 146 can be oriented substantially parallel to each other. Each leg 146 includes a hook 149 at its free end 151. The hooks 149 are configured to pass through at least a portion of the slot 140 and contact walls forming the slot 140 such that the mechanical stop 143 is coupled to the housing 128. In operation, the user can move the piston 134 in a direction away from the outlet control valve assembly 155 until a shoulder of the piston 134 contacts the mechanical stop 143. When the shoulder contacts the mechanical stop 143, the mechanical stop 143 prevents, or at least inhibits, the piston 134 from moving farther in a proximal direction (i.e., away from the outlet control valve assembly 155).

The injector 125 can further include a discharge check valve disposed between the fluid chamber 168 and the outlet control valve body 156 along the defined fluid flowpath. The discharge check valve can be configured operated in the manner as described above with respect to the discharge check valve. In the example shown, the outlet control valve body 156 and outlet control switch 171 are configured as a three-way stopcock valve and can additionally be configured in any alternative embodiment as previously discussed.

Referring now to Figs. 20 - 21E and 25A - 25F, the outlet control switch 171 includes a plug portion 198 that couples the outlet control switch 171 to the outlet control valve body 156 such that the outlet control switch 171 is rotatably seated within the outlet control valve body 156. In accordance with the illustrated embodiment, the outlet control valve assembly 155 defines a three (3) way stopcock, illustrated as a T-valve or Y-valve. In particular, as seen in Fig. 21E, the outlet control valve assembly 155 defines a valve inlet channel 201 and at least one valve outlet channel 204, for example two valve outlet channels 204, that extend into the valve body 156. In the embodiment as shown, the plug portion 198 includes multiple intersecting switch channels 207 that are in fluid communication with one another and that can be rotatably aligned to the valve inlet channel 201 and valve outlet channels 204 of the outlet control valve body 156 upon rotation of the outlet control switch 171. This rotatable configuration permits a bifurcation of the pressurized fluid flow; namely, the fluid can selectively enter all of the valve outlet channels, for example two of the valve outlet channels, or alternatively only one selected valve outlet channel, or yet further still none of the valve outlet channels depending upon the rotatable positioning of the outlet control switch. It should be appreciated that the outlet control valve assembly 155 can be configured to selectively supply pressurized fluid to more than two valve outlet channels 204, for instance when the injector 125 includes more than two outlet ports 159. For example, the injector 125 can include three, four, or more outlet ports 159, and the outlet control valve assembly 155 can be configured with an equivalent number of valve outlet channels 204. The outlet control switch 171, in turn, can be selectively aligned to supply pressurized fluid to one or more or none of the multiple outlet ports as desired.

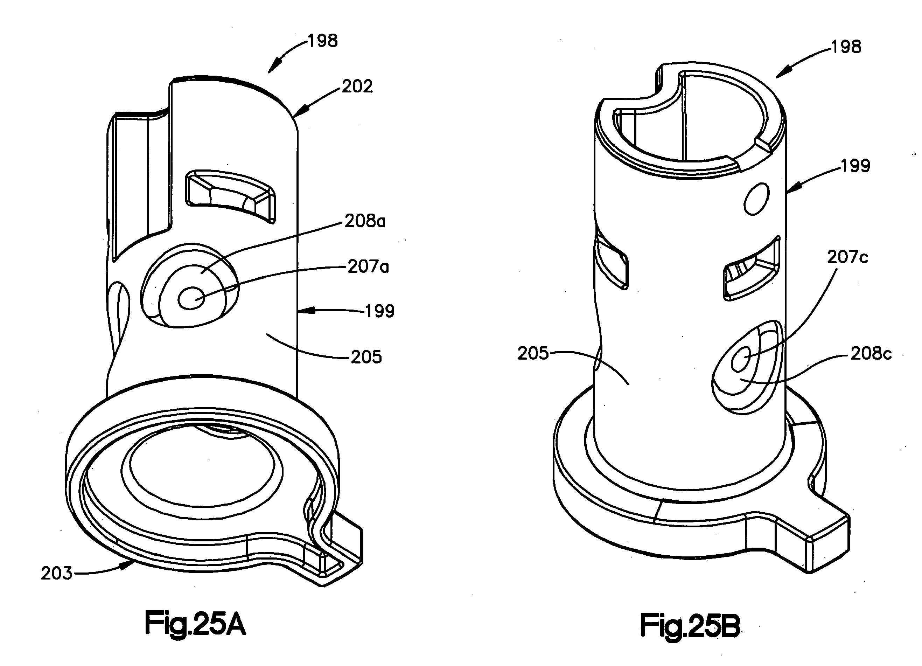

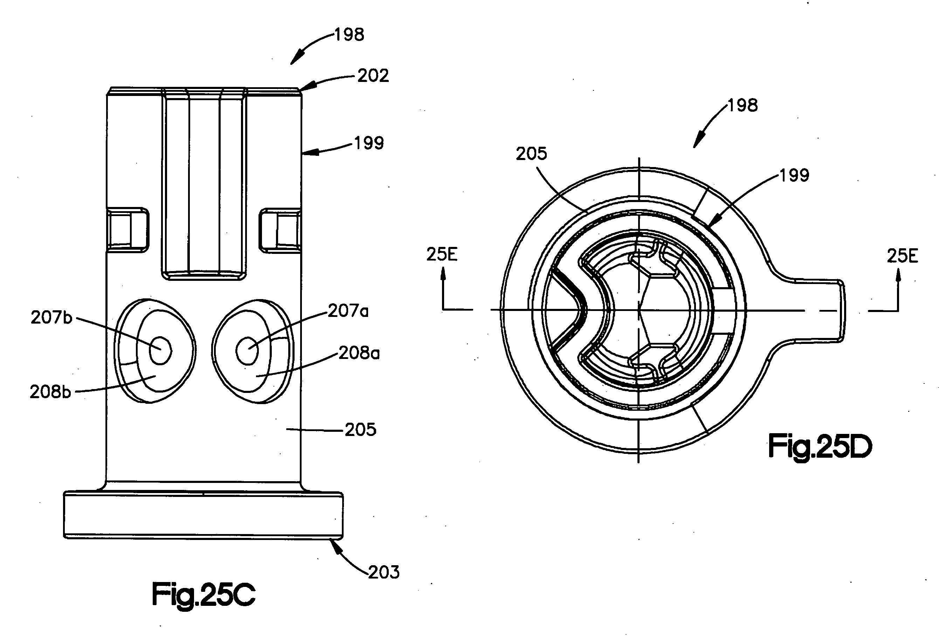

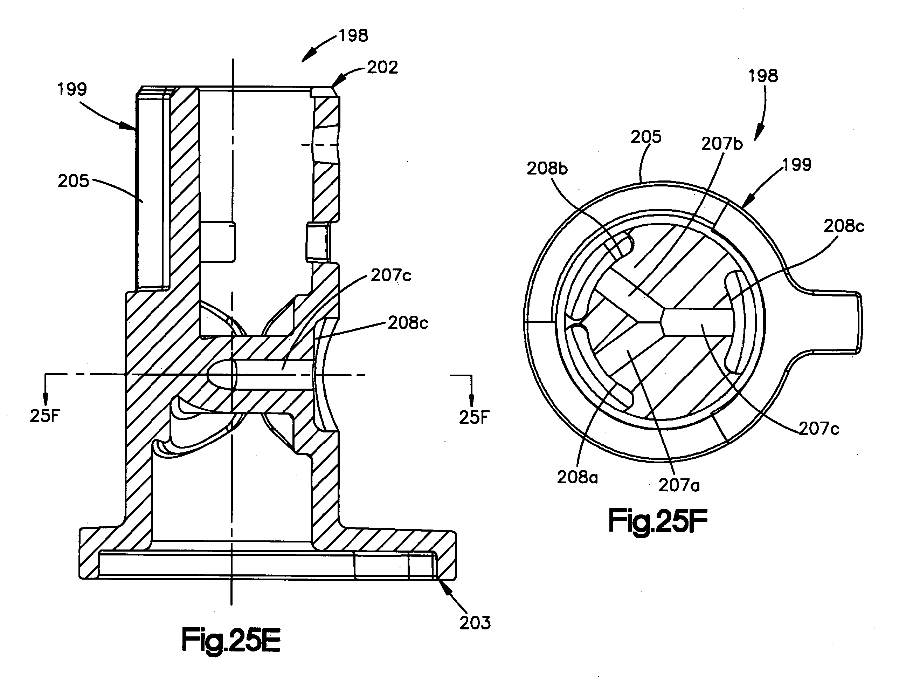

As shown in Figs. 25A-25F, an embodiment of the plug portion 198 includes a body 199 that defines a first or top end 202 and a second or bottom end 203. The body 199 defines an outer surface 205 that extends between the top end 202 and the bottom end 203. The plug portion 198 further defines a first switch channel 207a, a second switch channel 207b, and a third switch channel 207c. The first and second switch channels 207a and 207c are in fluid communication with the third switch channel 207c. Each of the first switch channel 207a, second switch channel 207b, and third switch channel 207c extends through the outer surface 205 and into the body 199 of the plug portion 199. The outer surface 205 defines a first recess 208a, a second recess 208b, and a third recess 208c each configured and sized to receive a fluid-tight seal 209 (Fig. 20), such as an O-ring. The fluid tight seal 209 can be a liquid-tight seal, and is configured to establish a fluid-tight connection (e.g., liquid-tight connection) between the valve body 156 (Fig. 20) and the plug portion 198. The first recess 208a is disposed on the outer surface 205 about an end of the first switch channel 207a. The second recess 208b is disposed on the outer surface 205 about an end of the second switch channel 207b. The third recess 208c is disposed on the outer surface 205 about an end of the third switch channel 207c..

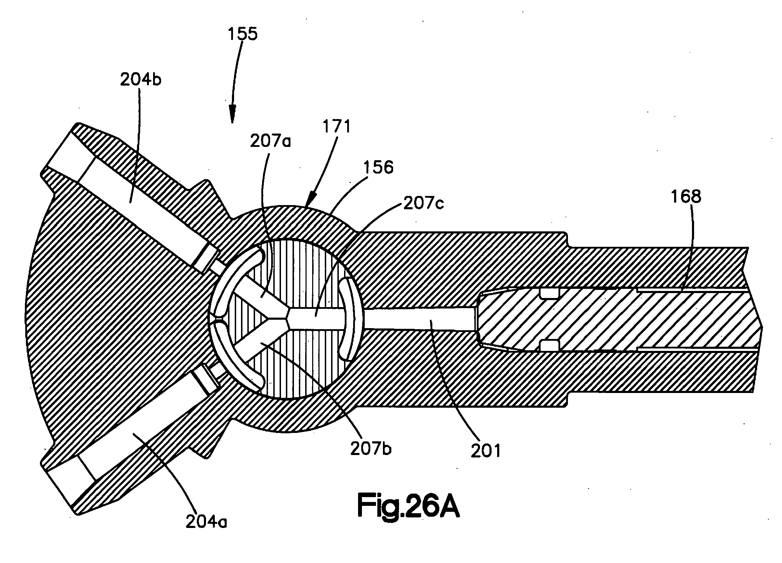

In the example shown in Fig. 26A, the control switch 171 is in a first position relative to the valve body 156 that causes the control valve assembly 155 to assume a fully open configuration. As discussed above, the switch channels 207 include a first switch channel 207a, a second switch channel 207b, and a third switch channel 207c. The first and second switch channels 207a and 207b are each in fluid communication with the third switch channel 207c. In the fully open configuration illustrated in Fig. 26A, the first switch channel 207a is aligned and in fluid communication with a first valve outlet channel 204a, the second switch channel 207b is aligned and in fluid communication with a second valve outlet channel 204b, and the third switch channel 207c is aligned and in fluid communication with the valve inlet channel 201. Thus, in the fully open configuration, fluid can flow from the valve inlet channel 201 to both valve outlet channels 204a and 204b.

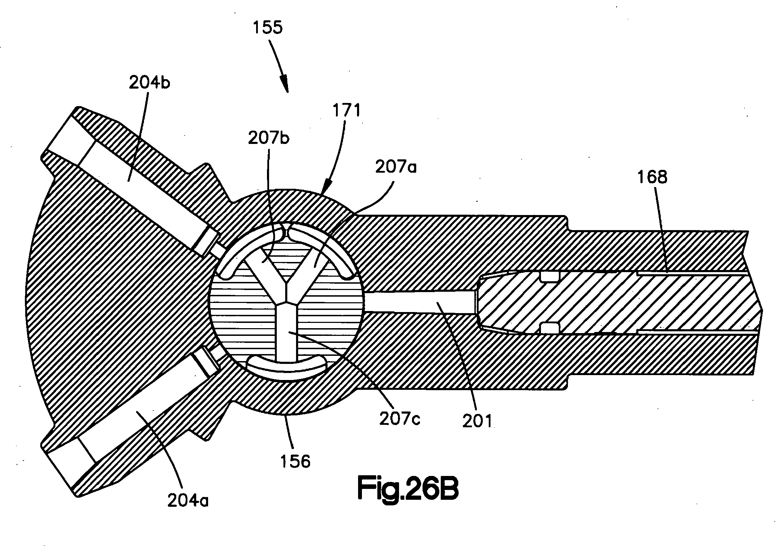

Referring to Fig. 26B, the control switch 171 is in a second position relative to the valve body 156 that causes the control valve assembly 155 to assume a fully closed configuration. In particular, the switch channels 207a-c are not aligned or in fluid communication with any of the valve inlet channel 201 or valve outlet channels 204a and 204b. Thus, in the fully closed configuration, fluid is prevented from flowing from the valve inlet channel 201 to the valve outlet channels 204a and 204b.

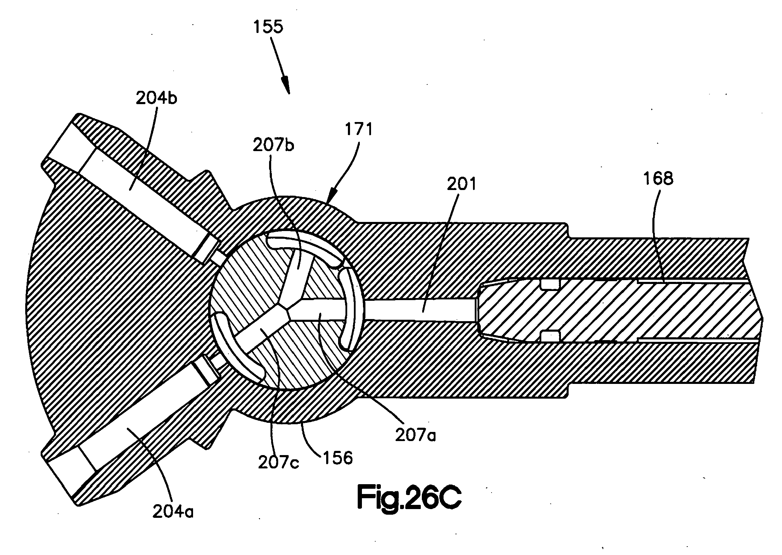

Referring to Fig. 26C, the control switch 171 is in a third position relative to the valve body 156 that causes the control valve assembly 155 to assume a first selectively open configuration. In particular, the first switch channel 207a is aligned and in fluid communication with the valve inlet channel 201, and the third switch channel 207c is aligned and in fluid communication with the second valve outlet channel 204b. However, the rotational positioning of the outlet control switch 171 is such that the second switch channel 207b is not aligned with, and is not in fluid communication with, the valve outlet channel 204a. In the first selectively open configuration, fluid can flow from the valve inlet channel 201 to the second valve outlet channel 204b, but not to the first valve outlet channel 204a.

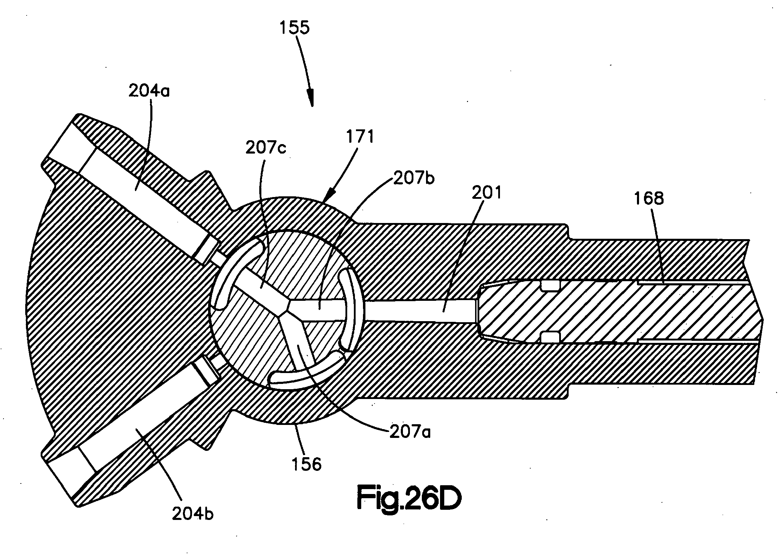

In the example shown in Fig. 26D, the control switch 171 is in a fourth position relative to the valve body 156 that causes the control valve assembly 155 to assume a second selectively open configuration that selectively places a different outlet channel 204 in fluid communication with the valve inlet channel 201. In particular, the second switch channel 207b is aligned and in fluid communication with the valve inlet channel 201, and the third switch channel 207c is aligned and in fluid communication with the first valve outlet channel 204a. However, the rotational position of the outlet control switch 171 is such that the first switch channel 207a and the valve outlet channel 204b are not in alignment and are not in fluid communication with each other. Thus, in the second selectively open configuration, fluid can selectively flow from the valve inlet channel 201 to the valve outlet channel 204a, but not to the valve outlet channel 204b. It should be appreciated that in the selectively open configurations, the valve body 156 closes and seals the second switch channel 207b such that fluid cannot flow from the valve inlet channel 201 and out the second switch channel 207b.

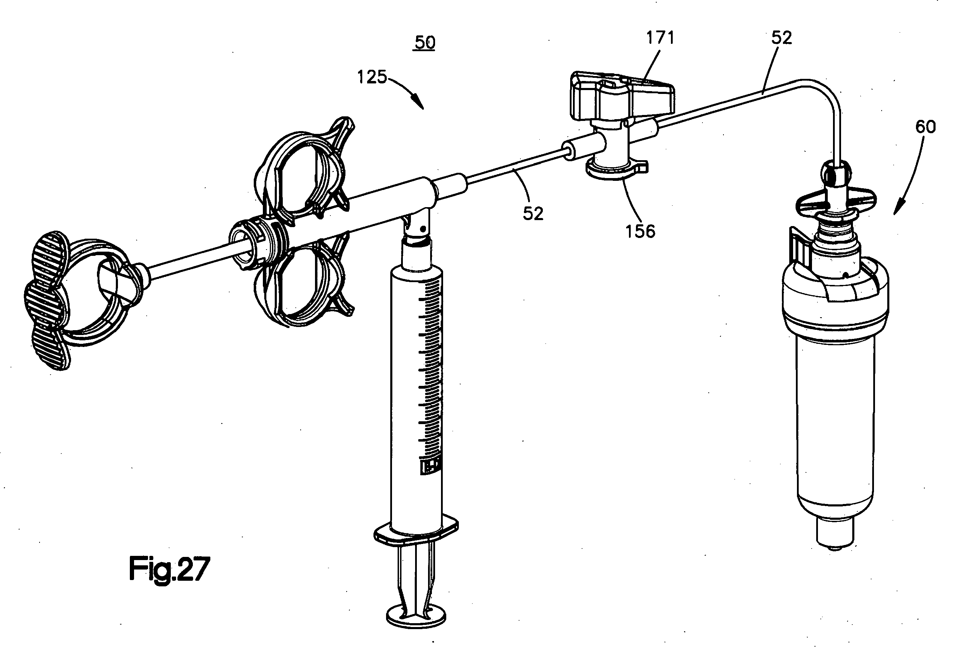

The outlet control valve body 156 and outlet control switch 171 can also be spaced from the fluid chamber 168 and housing 128 and connected via tubing 52 as shown in the embodiment in Fig. 27.

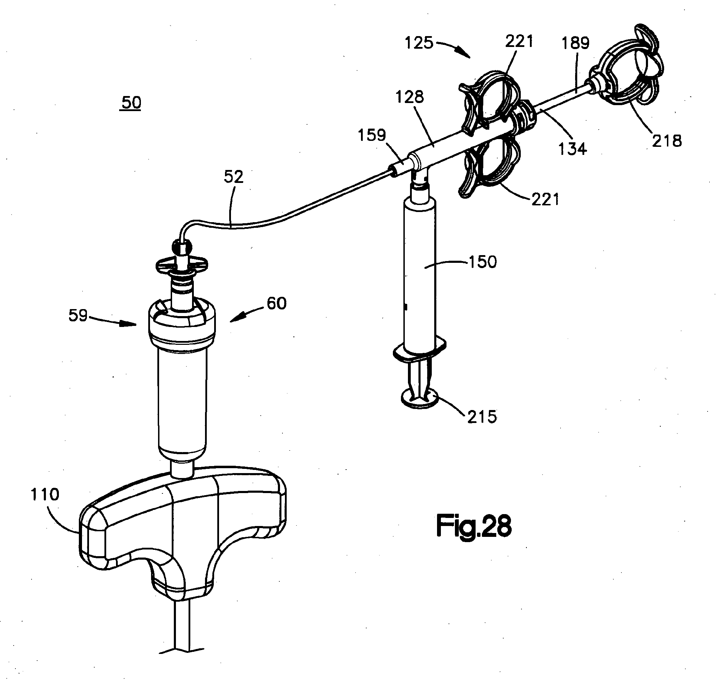

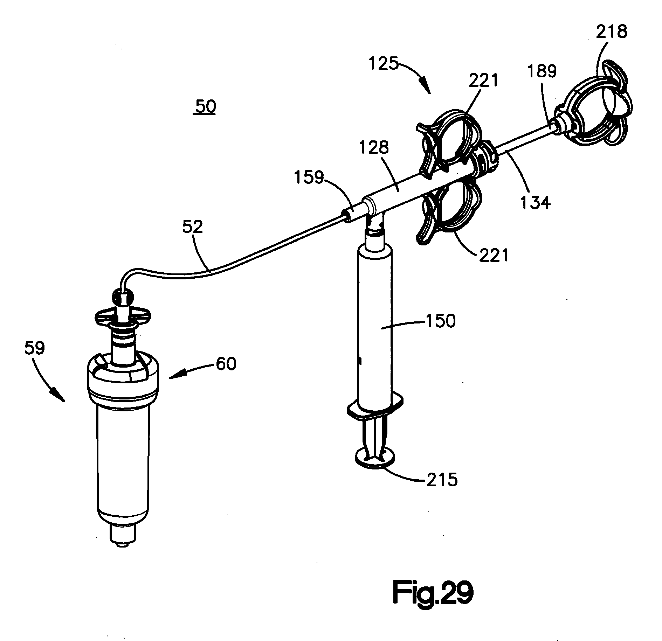

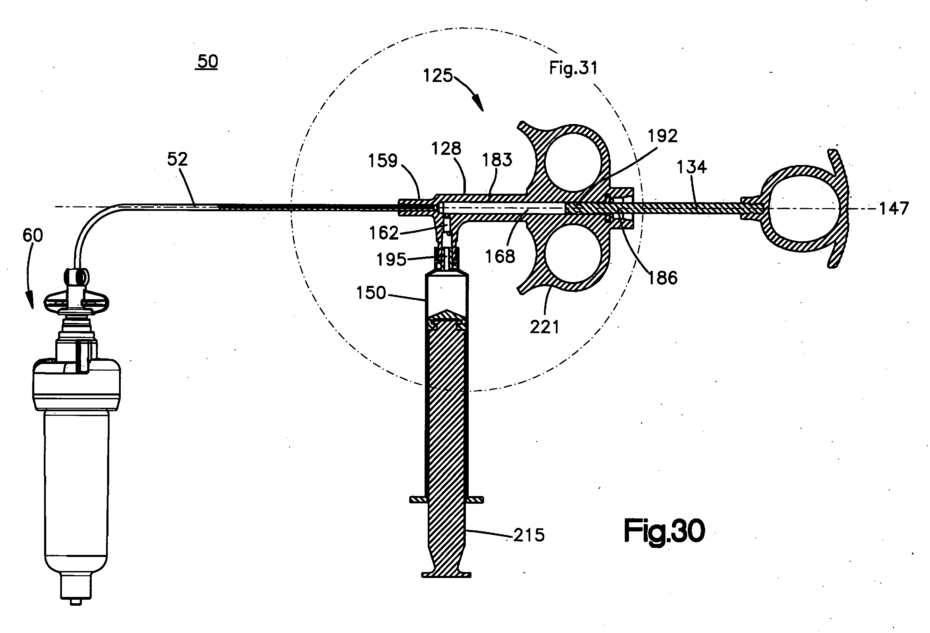

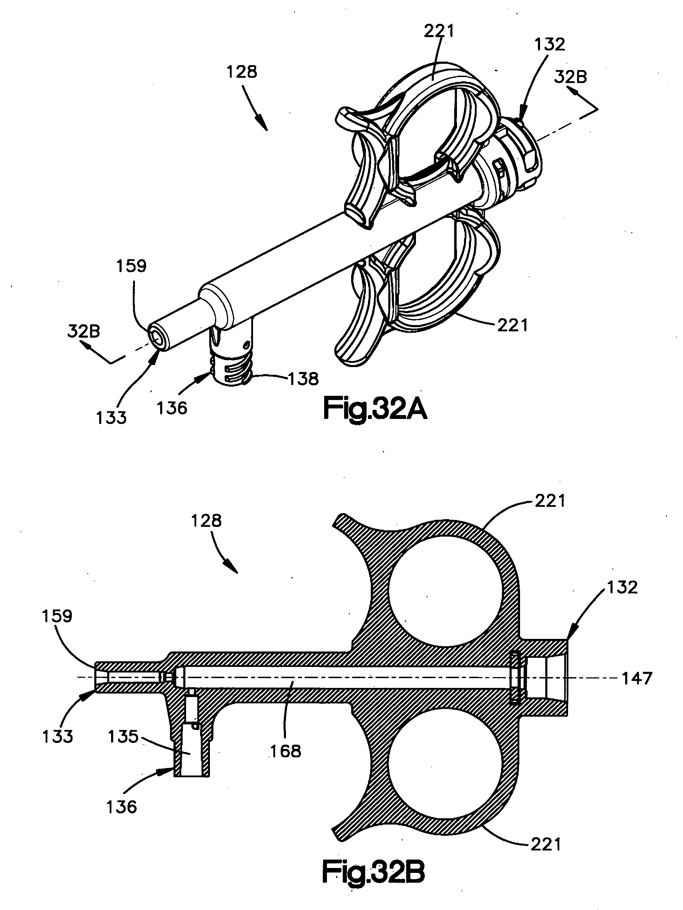

Referring now to Figs. 28-32B, the hydraulic injection system 50 can include a hydraulic injector 125 constructed in accordance with another embodiment and configured to deliver pressurized fluid to the cement cartridge 60 so as to deliver a bone cement housed in the cartridge body 63 into the cannulated needle 110 and ultimately to the target anatomical location. In accordance with the illustrated embodiment, the injector 125 includes an outer housing 128 that defines an interior fluid chamber 168 extending along a central axis 147. The outer housing 128 can be substantially syringe-shaped in accordance with the illustrated embodiment. The injector 125 further includes a fluid reservoir 150, a piston 134, and an outlet port 159. The injector 125 defines a fluid flowpath from the reservoir 150, through the fluid chamber 168, and finally through the outlet port 159.

The injector 125 further includes a suction check valve 162 disposed between the fluid reservoir 150 and the fluid chamber 168 along a fluid flowpath, and allows the hydraulic fluid to flow unidirectionally from the reservoir opening 195 in the fluid reservoir 150 into the fluid chamber 168. According to one embodiment, the suction check valve 162 is a low crack pressure valve such that the crack pressure of the suction check valve 162 is less than flow resistance of the pressurized fluid in the conduit 52 (i.e., backflow). For example, the low crack pressure valve can have a crack pressure in the range having a lower end substantially equal to or greater than approximately 0KPa and an upper range substantially equal to or less than approximately 100kPa, which can be substantially equal to or less than approximately 50kPa, for instance substantially equal to or less than approximately 20kPa, and in one embodiment substantially equal to or less than approximately 10kPa.

It should be appreciated that the crack pressure values expressed above can define a pressure differential between opposing sides of the check valve as has described above. For example, a check valve having a rating of over 100kPa can provide a low crack pressure check valve where a biasing element, for example a spring having a biasing force greater than 0kPa, is positioned so as to bias the reservoir plunger 215 and force fluid flow from the reservoir 150 through the suction check valve 162. In such an arrangement, the absolute pressure differential that causes the suction check valve to open is the difference between the valve rating of the suction check valve minus the biasing force of the spring. Thus, such a suction check valve would open at a crack pressure lower than its actual valve rating and operate as a low crack pressure check valve.

The reservoir 150 can be connected to the housing 128 by a fluid tight connection between the reservoir opening 195 and the suction check valve 162, for example via a Luer lock or alternative connection, and can be directly connected as shown in the example figures, or can alternatively be indirectly connected via tubing as has been previously described. Additionally, as has been previously described the reservoir 150 can be formed as an integral part of the housing 128.

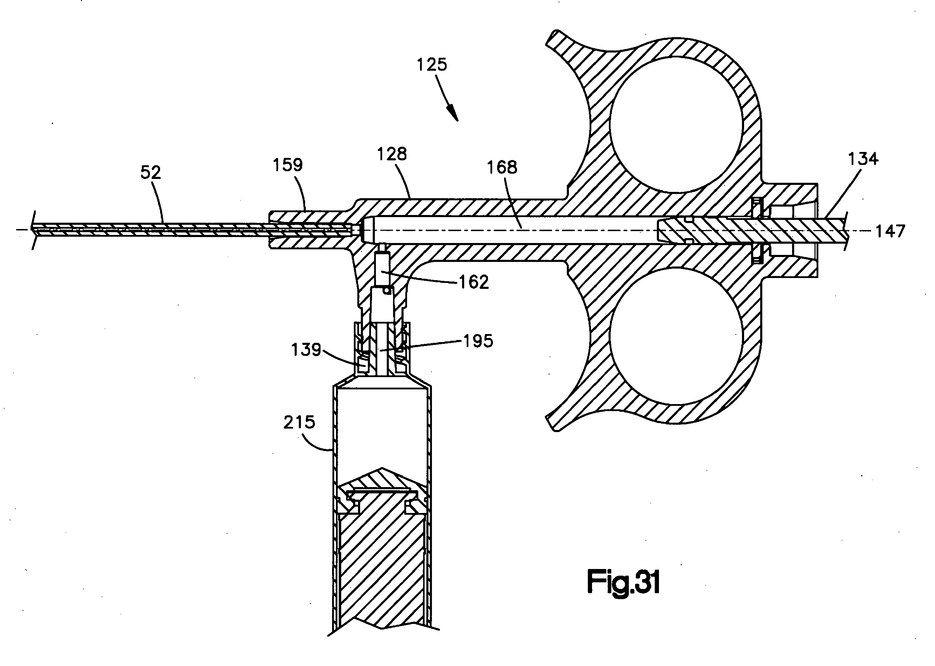

The housing 128 defines a first or proximal end 132 and a second or distal end 133. The fluid chamber 168 extends from the proximal end 132 to the distal end 133 along central axis 147. The housing 128 further defines a transverse channel 135 that can be substantially perpendicular to the central axis 147. The transverse channel 135 is configured and sized to receive the suction check valve 162, and is configured to establish fluid communication between the fluid reservoir 150 and fluid chamber 168. The housing 128 further comprises a reservoir fastener 136 disposed about at least a portion of the transverse channel 135. The reservoir fastener 136 can be, for example, a Luer lock connection, or any other fastener capable of fluidly coupling the fluid reservoir 150 with the fluid chamber 168. For instance, the reservoir fastener 136 can include external threads 138 configured to mate with inner threads 139 (Fig. 31) of the fluid reservoir 150.

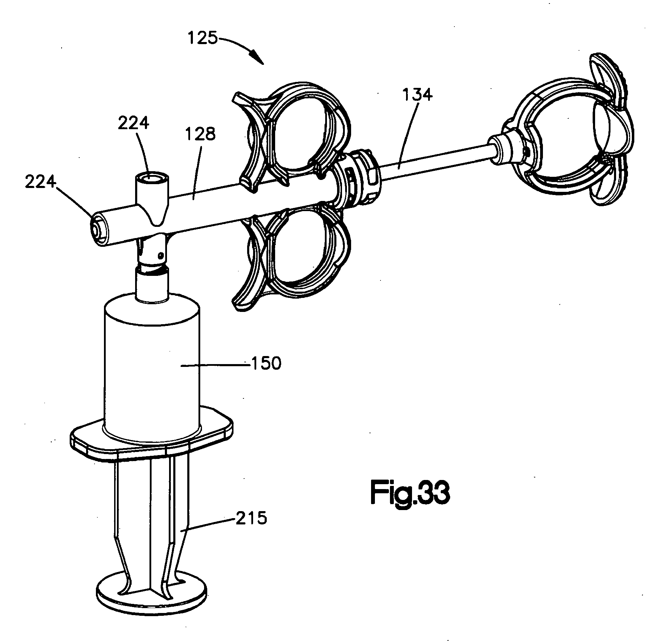

Referring also to Fig. 33, housing 128 of the injector 125 can also include one or more outlet ports 159 disposed adjacent respective universal connection mounts 224. The outlet ports 159 can be in fluid communication with the fluid chamber 168 in the manner described above. The connection mounts 224 can be adapted to connect with for example, a length of tubing, a pressure relief valve, or a pressure monitor gauge where desired, so as to place the outlet ports 159 in fluid-tight communication with the tubing. For example, where the injector 125 includes both a suction check valve and a discharge check valve, a pressure relief valve could permit the user to manually reduce or alternatively prevent the buildup of excessive pressures in the system and could additionally control the flow of cement out of the cement cartridge assembly as has been previously described above.

The hydraulic injector 125, as shown in Figs. 1-33, can operate generally as a syringe pump. The hydraulic injection system 50 can be primed in alternative fashions. For instance, the fluid reservoir 150 can be configured as a syringe, such that the reservoir plunger 215 is actuated to force fluid through the reservoir opening 195 and through the suction check valve 162 into the fluid chamber 168. Alternatively, the piston 134 can be withdrawn from the fluid chamber 168 in a suction stroke, causing the suction check valve 162 to open and pull hydraulic fluid through the reservoir opening 195 into the fluid chamber 168.

Alternatively, the injector 125 can be devoid of a discharge check valve, and the hydraulic injection system 50 can be primed with fluid prior to initiating the pressure stroke. The reservoir plunger 215 can be actuated to force fluid from the reservoir 150 into the fluid chamber 168 and into the outlet port(s) 159, and finally along to the cement cartridge 60 where the aeration venting valve 84 can be open to purge air and fluid from the hydraulic injection system 50. In further embodiments, the injector 125 can include an outlet control valve assembly 155, and the outlet control valve body 156 and the outlet control switch 171 can be aligned in an open position so as to allow fluid to flow from the fluid chamber 168 through the outlet control valve body 156 and the outlet control switch 171 into the outlet port(s) 159.

In accordance with one embodiment, the injector 125 can include a discharge check valve, such that repeated actuation of the piston 134 in alternating suction and pressure strokes, similar to the operation of the trigger based injector previously described above, allows the hydraulic fluid to move from the reservoir 150 through the hydraulic injection system 50. Repeated actuation of the piston 134 can also allow fluid to flow through the hydraulic injection system 50, for instance when the injector 125 includes a low crack pressure suction check valve 162, such that a suction stroke of the piston 134 causes the suction check valve 162 to open, thereby allowing the hydraulic fluid to flow from the reservoir to fill the hydraulic injection system 50, as opposed to pulling the hydraulic fluid from the hydraulic injection system 50 back into the fluid chamber 168.

Once fluid from the reservoir 150 fills the fluid chamber 168, the piston can be actuated in a pressure stroke that closes the suction check valve 162 preventing fluid from re-entering the reservoir 150 while simultaneously the fluid contained in the fluid chamber 168 is forced towards the outlet port(s) 159 or in certain embodiments as described herein, the outlet control valve body 156. Furthermore, as has been previously described, the injector 125 constructed in accordance with certain embodiments can include an outlet control valve assembly 155, such that the fluid flow can be selectively controlled at the outlet control valve body 156 by the positioning of the outlet control switch 171, and can flow through one or more of the valve outlet channels 204 as desired. Thus, the piston 134 can move with respect to the housing 128 between a first or proximal position and a second or distal position. When the piston 134 is moved from the first position to the second position (i.e., pressured stroke), hydraulic fluid in the fluid chamber 168 can be ejected out of the housing 128 via the outlet port(s) 159. In an embodiment, the movement of the piston 134 from the first position to the second position is a movement in a direction away from the outlet port(s) 159. When the piston 134 is moved from the second position to the first position (i.e., suction stroke), hydraulic fluid in the fluid reservoir travels toward the fluid chamber 168. In an embodiment, the movement of the piston 134 from the second position to the first position is a movement in a direction toward the outlet port(s) 159.

According to an embodiment, continued repeated actuation of the piston 134 maintains the pressurized fluid flow through the hydraulic injection system 50. According to another embodiment, for example where the hydraulic injection system 50 has been primed with fluid through actuation of the reservoir plunger 215, a single maintained pressure stroke of piston 134 maintains the pressurized fluid flow through the hydraulic injection system 50. Thus, it can be appreciated that pressurized fluid flow can be delivered to the cement cartridge assembly 59 through a single actuation of the piston 134, as well as, through multiple repeated actuations of the piston 134. The pressurized fluid ultimately reaches the cement cartridge assembly 59 as previously described thus providing a hydraulic force sufficient to move a quantity of bone cement from the cartridge 60 to the target anatomical location(s), for example, the vertebra of a spine in a bi-pedicular Vertebroplasty / cementoplasty procedure. Typically the amount of bone cement injected per actuation is in the order of 0.1 to 1ml. In an embodiment, the volume of the fluid chamber 168 of the hydraulic injector 125 can be significantly smaller than the volume of the internal cavity 96 of the cement cartridge assembly 59. As a result, in this embodiment, multiple repeated actuations of the piston 134 can be performed to eject all of the bone cement from the cement cartridge assembly 59.

A method for injecting bone cement to a target anatomical location can include one or more, up to all, of the following steps in any order as desired unless otherwise indicated. For instance, the method can include the step of filling an interior cavity of a cement cartridge with a bone cement, the step of connecting the cement cartridge to a cannulated needle that accesses a target anatomical location, the step of connecting the cement cartridge to a hydraulic injector via a length of tubing such that the injector can deliver a pressurized fluid to the cartridge through at least one outlet port located on the injector, the step of selectively moving an outlet control switch of the injector relative to an outlet control valve body such that pressurized fluid can be selectively directed from a fluid reservoir in the injector through the outlet control valve body and outlet control switch and through the at least one outlet port, the step of actuating a piston of the injector to force fluid from the fluid reservoir to the cement cartridge, and the step of hydraulically forcing bone cement from the cartridge through the cannulated needle to the target anatomical location.

A method for injecting bone cement to a target anatomical location can include one or more, up to all, of the following steps in any order as desired unless otherwise indicated. For instance, the method can include the step of introducing cement into an interior cavity of a cement cartridge, the step of connecting the cement cartridge to a cannulated needle that is configured to access a target anatomical location so as to place the interior cavity of the cement cartridge in fluid communication with the needle, the step of connecting the cement cartridge to a hydraulic injector via a conduit such that the injector can deliver a pressurized fluid to the cartridge through at least one of first and second outlet port of the injector, the step of actuating an outlet control valve assembly of the hydraulic injector so as to direct pressurized fluid can be selectively directed from the injector, through the outlet control valve assembly, and into at least a select one of the first and second outlet ports, and the step of actuating a piston of the injector to force fluid from the hydraulic injector to the cement cartridge, so as to hydraulically force bone cement from the cement cartridge through the cannulated needle to the target anatomical location. The step of actuating the piston can be repeated more than once. The method can further include the step of selectively moving a venting valve of the cement cartridge from a closed position to an open position to remove gases from the cement cartridge before actuating the piston.

A method for injecting bone cement to a target anatomical location can include one or more, up to all, of the following steps in any order as desired unless otherwise indicated. For instance, the method can include the step of filling an interior cavity of a cement cartridge with a bone cement, the step of connecting the cement cartridge to a cannulated needle that accesses a target anatomical location, the step of connecting the cement cartridge to a hydraulic injector via a length of tubing such that the injector can deliver a pressurized fluid to the cartridge through at least one outlet port located on the injector, the step of actuating a piston of the injector to force fluid from the fluid reservoir to the cement cartridge assembly, wherein the injector includes a suction check valve having a low crack pressure disposed between the piston and the fluid reservoir along a fluid flowpath, and the step of hydraulically forcing bone cement from the cartridge through the cannulated needle to the target anatomical location.

The method can further include a repeated step of actuating the piston, such that the low crack pressure of the suction check valve allows additional fluid from the fluid reservoir to enter one or more cement cartridges.

A method for injecting bone cement to a target anatomical location can include one or more, up to all, of the following steps in any order as desired unless otherwise indicated. For instance, the method can include the step
introducing a bone cement into an interior cavity of a cement cartridge, the step of connecting the cement cartridge to a cannulated needle that accesses a target anatomical location, the step of connecting a first end of the cement cartridge to a hydraulic injector that has a fluid reservoir, such that the interior cavity of the cement cartridge is in fluid communication with the fluid reservoir via a conduit such that the injector can deliver a pressurized fluid from the fluid reservoir to the interior cavity through the conduit, the step of connecting a second end of the cement cartridge to a cannulated needle that is configured to access a target anatomical location, so as to place the interior cavity of the cement cartridge in fluid communication with the needle, the step of actuating the injector to force fluid from the fluid reservoir to the interior cavity, wherein the injector includes a suction check valve disposed between the piston and the fluid reservoir that prevents the pressurized fluid from traveling into the fluid reservoir; and the step of causing the fluid from the fluid reservoir to act against the cement cartridge so as to hydraulically force bone cement from the cartridge through the cannulated needle to the target anatomical location. The step of actuating the piston can be repeated more than once. The method can further include selectively moving a venting valve of the cement cartridge from a closed position to an open position to remove gases from the cement cartridge before actuating the piston.

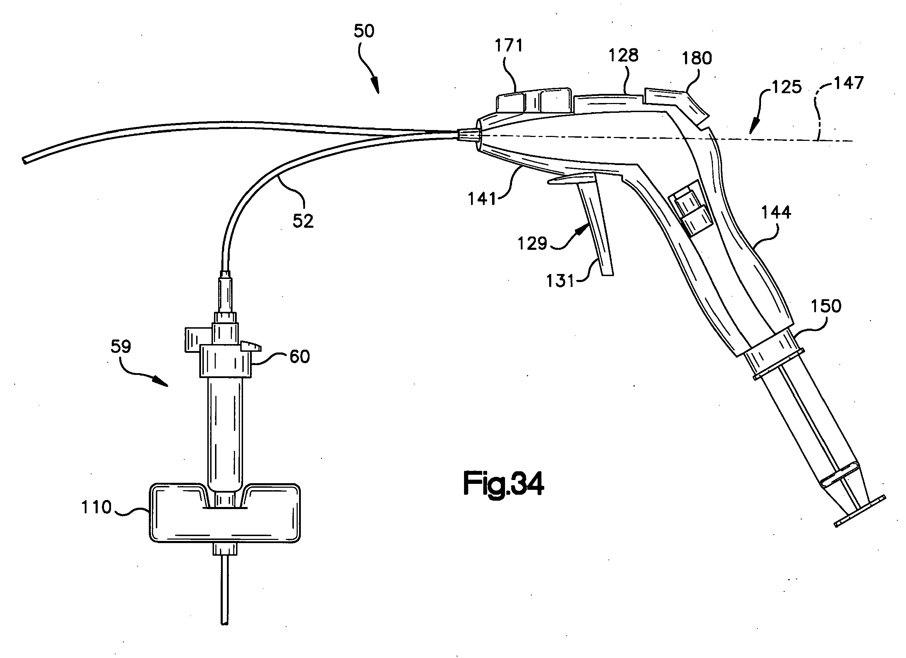

Referring now to Fig. 34, the hydraulic injector 125 is configured to deliver pressurized fluid to the cartridge 60. The pressurized fluid can have a hydraulic force sufficient to eject a quantity of bone cement, including both low and high viscosity bone cement, from the cartridge 60 into a cannulated needle 110 as described above. In accordance with the illustrated embodiment, the injector 125 includes an outer housing 128 shaped as gun or pistol having a barrel portion 141 that is elongate axially along a central axis 147, and a handle portion 144 extending substantially from the barrel portion 141 along a direction that is angularly offset with respect to the central axis 147. The hydraulic injector 125 further includes a trigger 129 that is respect to the central axis 147. The hydraulic injector 125 further includes a trigger 129 that is pivotally attached to the barrel portion 141 of the housing 128 such that a trigger arm 131 of the trigger 129 extends below the barrel portion 141 and is capable of movement (e.g., translation) along a direction having an axial directional component parallel to the central axis 147.

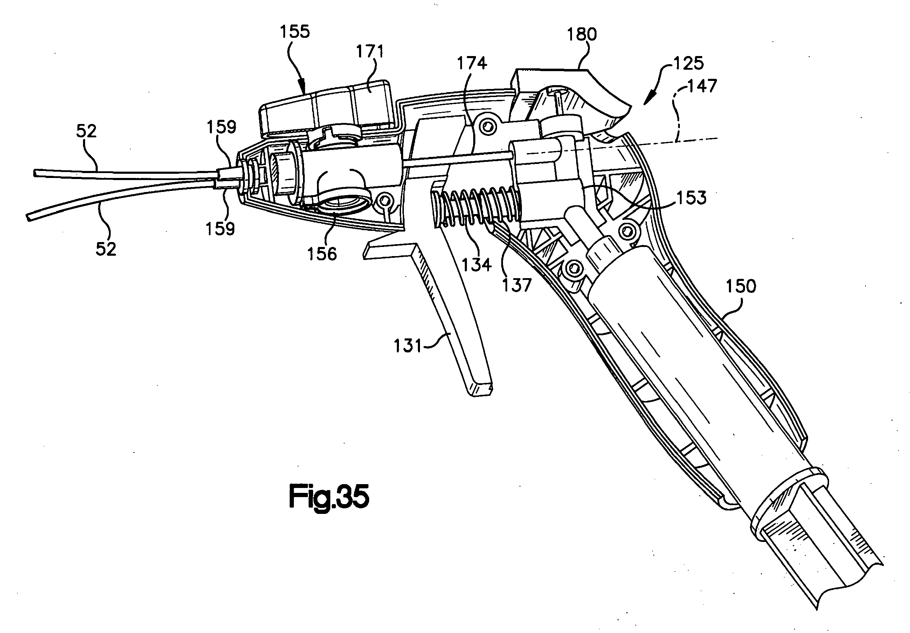

Referring now to Figs. 35-36, the injector 125 also includes a fluid reservoir 150, a pressure chamber 153 coupled to the fluid reservoir 150 and in fluid communication with the fluid reservoir 150, and a piston 134 operably coupled between the trigger 129 and the pressure chamber 153. The injector 125 can further include an outlet control valve assembly 155 in fluid communication with the pressure chamber 153. The control valve assembly 155 includes an outlet control valve body 156 and an outlet control switch 171 movably coupled to the outlet control valve body 156. The control valve assembly 155 further includes at least one outlet port 159, such as a pair of outlet ports 159, which are carried by the control valve body 156. The outlet control switch 171 can be moved between a plurality of positions that configures the valve assembly 155 to selectively supply a pressurized fluid through one or more of the outlet ports 159, or to block inlet fluid from flowing into either of the outlet ports 159. Accordingly, the user is able to selectively control the pressurized fluid through a particular outlet port or ports 159 as desired. In the example shown, the injector 125 includes two outlet ports 159, and the user can bifurcate the pressurized fluid flow through one, both, or none of the outlet ports 159, by adjusting the positioning of the outlet control switch 171. The pressurized fluid that flows from the outlet port(s) 159 is able to travel through tubing 52 to cartridge 60 where it is able to exert a force sufficient to push or expel a quantity of bone cement from cartridge 60.

It should be appreciated that the injector 125 can also be configured with more or less than two outlet ports 159 as shown in the drawings. For example, the injector 125 can define more than two outlet ports 159 such as three or four outlet ports 159, or any number as desired within the scope of the disclosure. It is contemplated within the scope of the disclosure that the injector 125 can contain as many as six outlet ports 159. The hydraulic injector 125 can be devoid of the control valve assembly 155, for instance when the hydraulic injector 125 defines a single outlet port 159 and selective fluid flow provided by the outlet control valve assembly 155 is not desired. Alternatively, when the hydraulic injector 125 defines a single outlet port 159, the hydraulic injector 125 can further include the outlet control assembly 155 which can be configured to selectively prevent or allow fluid flow to the single outlet port 159 from the pressure chamber 153.

With continuing reference to Figs. 35-36, the injector 125 defines a fluid flow path from the reservoir 150, through the pressure chamber 153 to the outlet control valve assembly 155. The fluid flow path further includes a selective fluid flow path portion defined by the outlet control valve assembly 155 through the outlet port(s) 159. The pressure chamber 153 includes a suction check valve 162 and a discharge check valve 165, and defines a piston retention bore 168 that is configured to slidably receive at least a portion of the piston 134. In particular, the piston retention bore is defmed by an inner surface 183 of the pressure chamber 153. The retention bore 168 extends axially into the pressure chamber 153, and can terminate within the pressure chamber 153 as illustrated. The piston 134 defines a first end 186 that is at least partially disposed within the piston retention bore 168. The first piston end 186 includes at least one piston seal 192, for example an O-ring, circumferentially disposed about an outer periphery of the piston 134 at the first piston end 186, and is configured to engages the inner surface 183 so as to define a fluid tight seal. The piston 134 can extend out from the piston retention bore 168, and in particular defines a second piston end 189 that is adapted to contact the trigger 131. The injector 125 further includes a biasing member, such as a coil spring 137 that surrounds at least a portion of the piston 134 between the trigger 131 and a portion of the pressure chamber 153, and can further surround at least a portion of the pressure chamber 153.

The suction check valve 162 is disposed between the fluid reservoir 150 and the fluid chamber 168 along the defined fluid flowpath and enables a one-way flow of fluid along a direction from a reservoir opening 195 in the fluid reservoir 150 toward, and to, the fluid chamber 168. The reservoir opening 195 is in fluid tight communication with the suction check valve 162, for example via a Luer lock connection, and can be directly connected as shown in the example figures, or can alternatively be indirectly connected via internal tubing. Similarly, the discharge check valve 165 is disposed between the fluid chamber 168 and the outlet control valve body 156 along the defined fluid flowpath and enables a one way flow of fluid from the fluid chamber 168 along a direction toward, and to, the outlet check valve body 156. In accordance with the illustrated embodiment, the discharge check valve 165 is in fluid communication with, and in particular in fluid tight communication with, the outlet control valve body 156 via internal tubing 174 that is connected between the discharge check valve 165 and the outlet control valve body 156. Alternatively, the discharge check valve 165 can be directly connected to the outlet control valve body 156. In the examples as shown, the discharge check valve 165 and the suction check valve 162 are configured as ball check valves that are known in the art. It should be appreciated that any suitable check valve can be used as the suction check valve or discharge check valve as desired.

The injector 125 can further include a pressure relief valve 177 disposed along the defined fluid flowpath between the discharge check valve 165 and the outlet control valve body 156. In accordance with the illustrated embodiment, the pressure relief valve 177 is disposed in the pressure chamber 153 as a ball check valve known in the art. A manual relief switch 180 can be connected to the pressure relief valve 177 such that actuation of the switch 180 by a user can open the pressure relief valve 177, thus venting and de-pressurizing the pressure chamber 153. The pressure relief valve 177 functions to allow the user control of the pressurized fluid being delivered to the cement cartridges 60 as previously described. For example, the user can actuate the switch 180 to enable a rapid loss of pressure in the system that can subsequently slow or even stop the flow of bone cement from the previously described cement cartridge assembly 59. As such, the user is able to reliably control the flow of bone cement to a target anatomical location through use of the manual relief switch 180 and pressure relief valve 177.

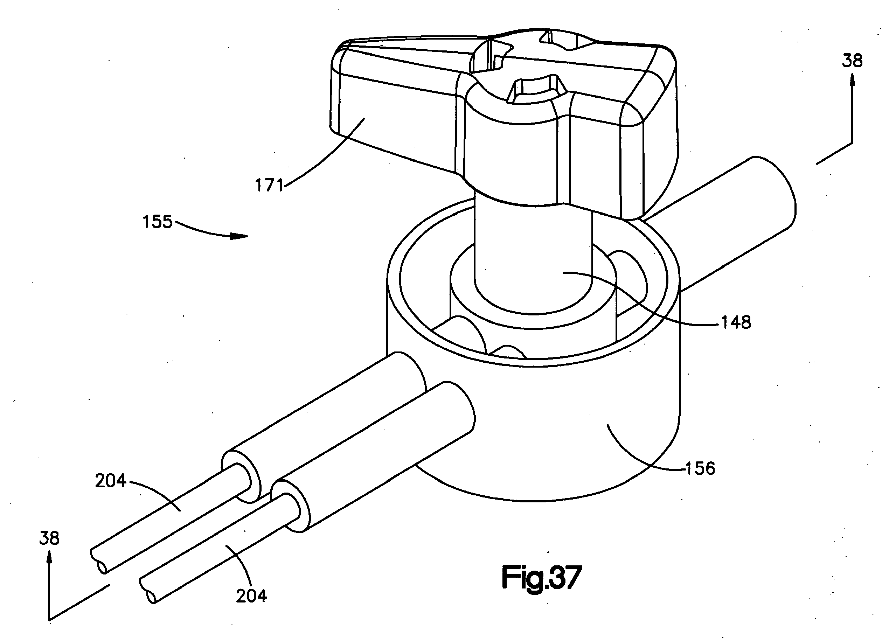

Referring now to Figs. 37 - 41, the outlet control switch 171 includes a plug portion 198 that couples the outlet control switch 171 to the outlet control valve body 156 such that the outlet control switch 171 is rotatably seated within the outlet control valve body 156. In accordance with the illustrated embodiment, the outlet control valve assembly 155 defines a three (3) way stopcock, illustrated as a T-valve or Y-valve. In particular, the outlet control valve assembly 155 defines a valve inlet channel 201 and at least one valve outlet channel 204, for example two valve outlet channels 204, which extend into the valve body 156. In the embodiment as shown, the plug portion 198 includes multiple intersecting switch channels 207 that are in fluid communication with one another and that can be rotatably aligned to the valve inlet channel 201 and valve outlet channels 204 of the outlet control valve body 156 upon rotation of the outlet control switch 171. This rotatable configuration permits a bifurcation of the pressurized fluid flow; namely, the fluid can selectively enter all of the valve outlet channels, for example two of the valve outlet channels, or alternatively only one selected valve outlet channel, or yet further still none of the valve outlet channels depending upon the rotatable positioning of the outlet control switch. It should be appreciated that the outlet control valve assembly 155 can be configured to selectively supply pressurized fluid to more than two valve outlet channels 204, for instance when the injector 125 includes more than two outlet ports 159. For example, the injector 125 can include three, four, or more outlet ports 159, and the outlet control valve assembly 155 can be configured with an equivalent number of valve outlet channels 204. The outlet control switch 171, in turn, can be selectively aligned to supply pressurized fluid to one or more or none of the multiple outlet ports as desired.

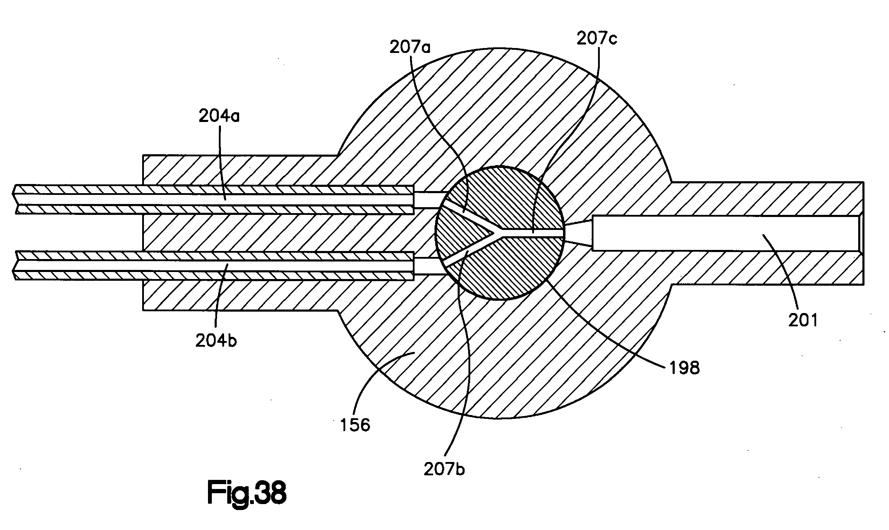

In the example shown in Fig. 38, the control switch 171 is in a first position relative to the valve body 156 that causes the control valve assembly 155 to assume a fully open configuration. The switch channels 207 include a first switch channel 207a, a second switch channel 207b, and a third switch channel 207c. The first and second switch channels 207a and 207b are each in fluid communication with the third switch channel 207c. In the fully open configuration illustrated in Fig. 38, the first switch channel 207a is aligned and in fluid communication with a first valve outlet channel 204a, the second switch channel 207b is aligned and in fluid communication with a second valve outlet channel 204b, and the third switch channel 207c is aligned and in fluid communication with the valve inlet channel 201. Thus, in the fully open configuration, fluid can flow from the valve inlet channel 201 to both valve outlet channels 204a and 204b.

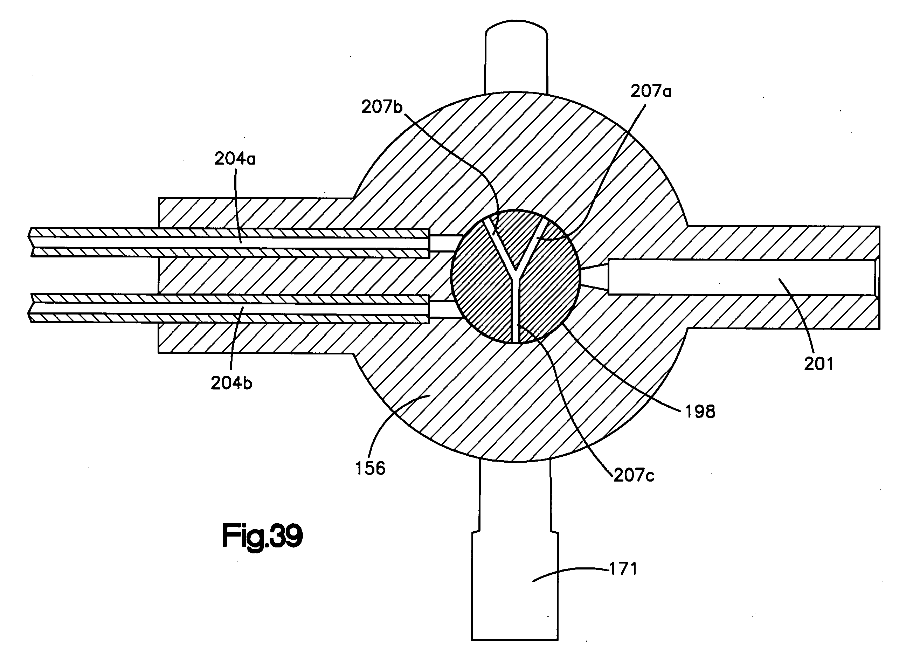

Referring to Fig. 39, the control switch 171 is in a second position relative to the valve body 156 that causes the control valve assembly 155 to assume a fully closed configuration. In particular, the switch channels 207a-c are not aligned or in fluid communication with any of the valve inlet channel 201 or valve outlet channels 204a and 204b. Thus, in the fully closed configuration, fluid is prevented from flowing from the valve inlet channel 201 to the valve outlet channels 204a and 204b.

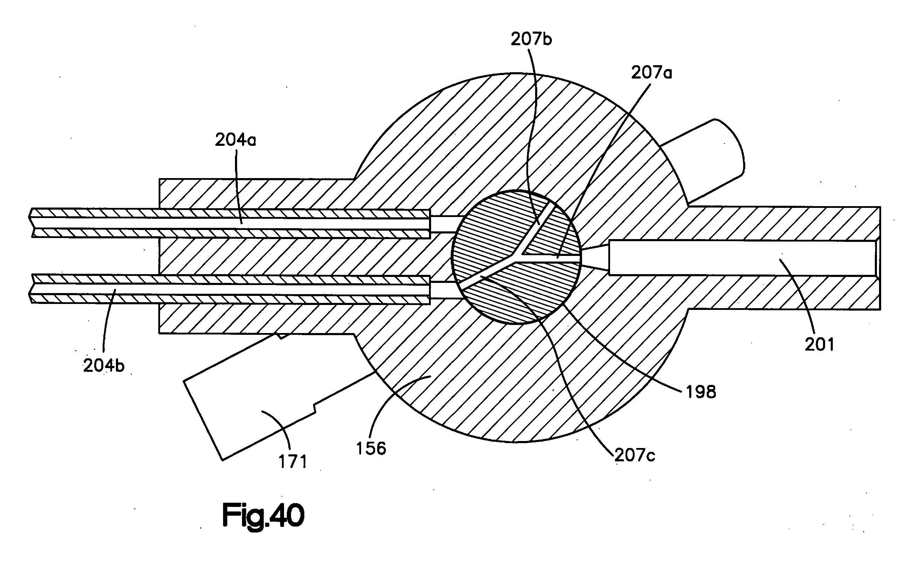

Referring to Fig. 40, the control switch 171 is in a third position relative to the valve body 156 that causes the control valve assembly 155 to assume a first selectively open configuration. In particular, the first switch channel 207a is aligned and in fluid communication with the valve inlet channel 201, and the third switch channel 207c is aligned and in fluid communication with the second valve outlet channel 204b. However, the rotational positioning of the outlet control switch 171 is such that the second switch channel 207b is not aligned with, and is not in fluid communication with, the valve outlet channel 204a. In the first selectively open configuration, fluid can flow from the valve inlet channel 201 to the second valve outlet channel 204b, but not to the first valve outlet channel 204a.

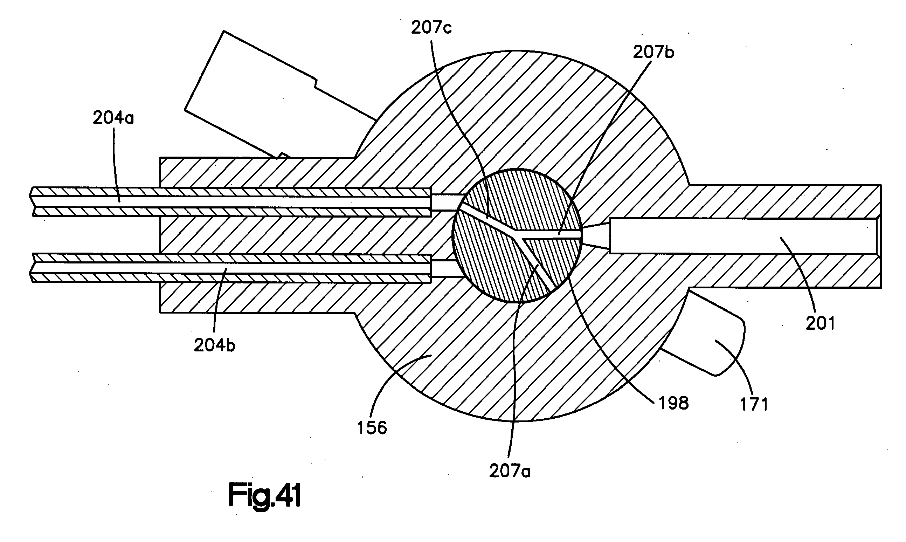

In the example shown in Fig. 41, the control switch 171 is in a fourth position relative to the valve body 156 that causes the control valve assembly 155 to assume a second selectively open configuration that selectively places a different outlet channel 204 in fluid communication with the valve inlet channel 201. In particular, the second switch channel 207b is aligned and in fluid communication with the valve inlet channel 201, and the third switch channel 207c is aligned and in fluid communication with the first valve outlet channel 204a. However, the rotational position of the outlet control switch 171 is such that the first switch channel 207a and the valve outlet channel 204b are not in alignment and are not in fluid communication with each other. Thus, in the second selectively open configuration, fluid can selectively flow from the valve inlet channel 201 to the valve outlet channel 204a, but not to the valve outlet channel 204b. It should be appreciated that in the selectively open configurations, the valve body 156 closes and seals the second switch channel 207b such that fluid cannot flow from the valve inlet channel 201 and out the second switch channel 207b.

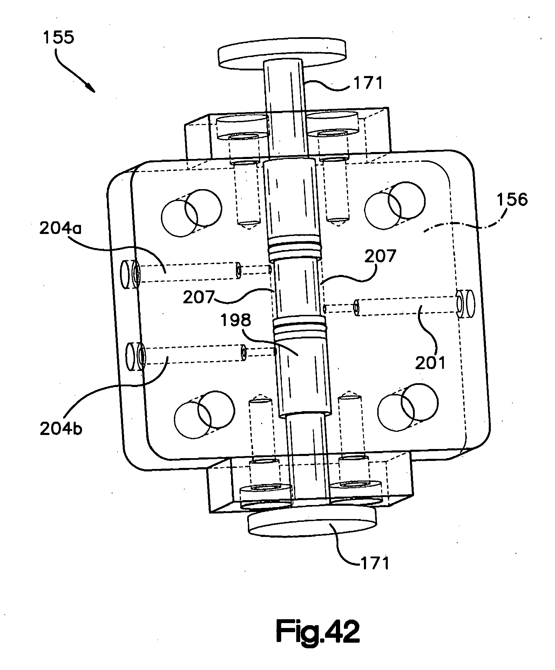

In an alternative embodiment, the control valve assembly 155 can alternatively be configured such that the plug portion 198 is slidably seated in the outlet control valve body 156. Accordingly, the outlet control switch 171 can be translated so as to iterate the control valve assembly 155 between the fully opened configuration, the fully closed configuration, and the first and second selectively open configurations. As illustrated in Fig. 42, the valve inlet channel 201 extends substantially parallel to the valve outlet channels 204a and 204b. The plug portion 198 includes at least one switch channel 207, for example two switch channels 207, and is seated perpendicular to and between the valve inlet channel 201 and the valve outlet channels 204. The outlet control switch 171 has a linear range of motion perpendicular to the alignment of the valve inlet channel 201 and valve outlet channels 204 such that movement of the outlet control switch 171 causes a corresponding relative movement of the plug portion 198 with respect to the valve inlet channel 201 and the valve outlet channels 204. As shown in Fig. 42, the plug portion 198 is aligned to allow fluid flow from the valve inlet channel 201, through the switch channels 207 and into the first valve outlet channel 204a. Additionally, this particular configuration prevents fluid flow to the second valve outlet channel 204b. It should be appreciated that, while not shown, the outlet control switch 171 can selectively move in either direction permitting the plug portion 198 to correspondingly move to engage both of the valve outlet channels 204a and 204b, or alternatively neither of the outlet channels 204a and 204b, depending upon the relative positioning of the plug portion 198 within the outlet control valve body 156. Thus, the fluid flow can be selectively bifurcated such that the pressurized hydraulic fluid can be directed through the outlet port(s) of the hydraulic injector to one or more of the cement cartridge assemblies described above.

Furthermore, the outlet control valve assembly 155 can define a pressure release valve that is configured to halt pressurized fluid flow to the cement cartridge assembly during operation of the injector. During operation, when pressurized hydraulic fluid flow is selectively channeled into any one or more of the valve outlet channels 204 as previously described, a user can immediately stop the pressurized fluid flow to a select one or more of the valve outlet channels 204. For instance, the outlet control switch 171 can be positioned so as to discontinue the flow of the pressurized hydraulic fluid to the select one or more of the valve outlet channels 204, thereby preventing hydraulic fluid flow to the cement cartridges operably coupled to the select one or more of the valve outlet channels 204. This can have the same effect as previously described above with respect to the pressure relief valve 177 and manual release switch 180.

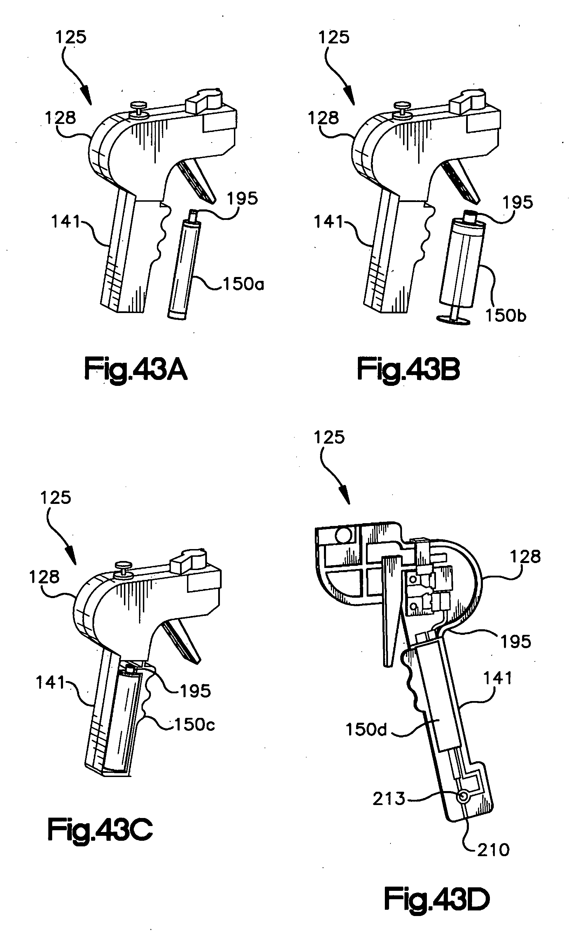

Fluid used in the operation of the hydraulic injection system 50 can be supplied by the fluid reservoir 150. The fluid reservoir 150 can be configured as desired such that the fluid reservoir opening 195 is in fluid tight communication with the suction check valve as previously described. Referring to Fig. 43A, in one embodiment, the fluid reservoir 150a can be a pre-filled sealed container separate from the housing 128. In such an embodiment, the reservoir 150a is configured to be insertable within the barrel portion 141 of the injector 125 and connect in fluid tight communication with the pressure chamber as previously described. Referring to Fig. 43B, in another embodiment, the fluid reservoir 150b can be a common medical syringe that can be filled with a suitable fluid at the time of use. In such an embodiment, the reservoir 150b is configured to be insertable within the barrel portion 141 of the injector 125 and connect in fluid tight communication with the pressure chamber as previously described. Referring to Fig. 43C, in a further embodiment, the fluid reservoir 150c, can be a pre-filled container that is an integral component of the injector 125 and housed within the barrel portion 141 and connected in fluid tight communication with the pressure chamber as previously described. Referring to Fig. 43D, in still a further embodiment, the fluid reservoir 150d can be an integral component of the injector 125, and housed within the barrel portion 141. For example, the fluid reservoir 150d can be monolithically formed with the barrel portion 141. In the embodiment as shown in Fig. 43D, the barrel portion 141 includes a reservoir port 210 that opens to the outside environment, and a reservoir valve 213 disposed along a fluid flow path between the reservoir port 210 and the fluid reservoir 150d. The reservoir port 210 can be configured as a common Luer lock that can connect with most commonly available medical syringes. The reservoir valve 213 can open and close controlling the fluid flow between the reservoir port 210 and the reservoir 150d. When the reservoir valve 213 is opened, fluid can be injected into or drained from the reservoir 150d via the reservoir port. Additionally, closure of the reservoir valve 213 prevents fluid leaking from the reservoir 150d during operation and ensures pressure integrity in the system. It should be appreciated that the reservoirs illustrated in Figs 43A and 43B can be designed to enable a surgeon or physician to use any available syringe (or other available sterile reservoir sources such as a pouch or bag like those commonly used to supply IV fluids) and fluid volume as desired when using the hydraulic injection system.

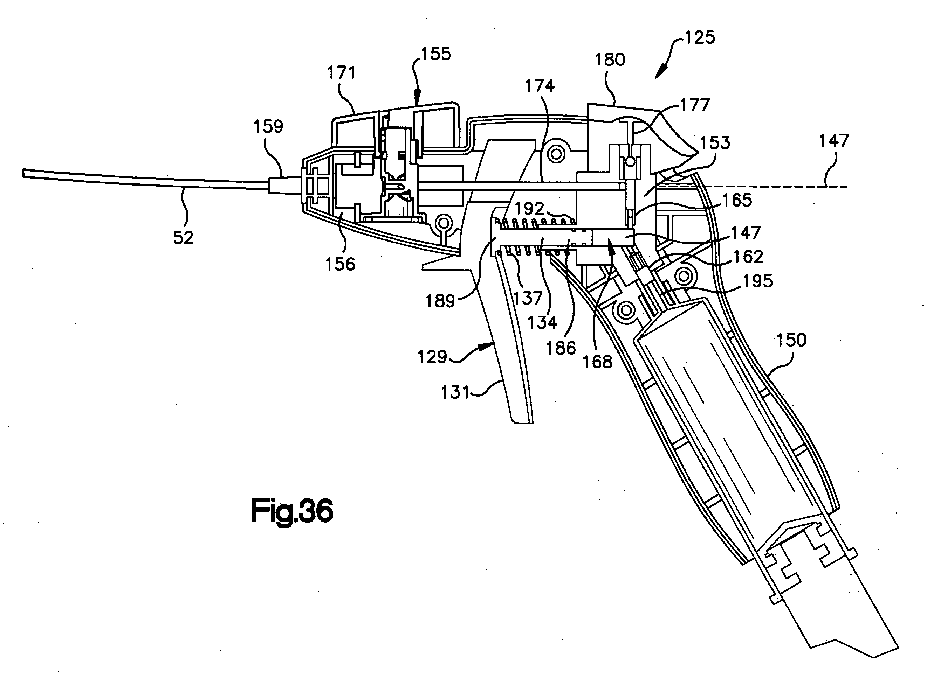

Referring again to Fig. 36, the hydraulic injector 125 can be configured as a trigger pump. As has been previously described, the injector 125 includes a suction check valve 162 and can further include a discharge check valve 165. The suction check valve 162 is configured to permits hydraulic fluid flow in only one direction, such as from the fluid reservoir 150 into the pressure chamber 153, and to prevent fluid flow from the pressure chamber 153 to the fluid reservoir. Likewise, the discharge check valve 165 can also permit hydraulic fluid flow in only one direction, such as from the pressure chamber 153 to either the outlet port(s) 159 or the outlet control valve assembly 155, where the outlet control valve assembly 155 is included. The discharge check valve 154 is configured to prevent hydraulic fluid flow from the outlet port(s) 159 to the pressure chamber 153, or the outlet control assembly 155.

The general principles of operation of check valves are well known within the art. In particular, check valves operate on a general principle of a pressure differential being generated between opposing sides of the check valve. Check valves typically remain closed and are able to seal off a potential fluidflow pathway. The check valves are calibrated to open once a sufficient pressure differential exists in order to equalize the pressure differential between the opposing sides and then reseal once the pressure differential drops below the calibrated setting of the check valve. Check valves can be designed to be "one-way" in that they can permit fluid flow through the valve in one direction but prevent a backflow of fluid in the opposite direction. The point or range at which a check valve is calibrated to open or seal is known in the art as the "crack pressure."

The trigger 131 as shown is in a first neutral position. In a first pressure stroke, the trigger 131 is actuated from the neutral position and contacts the second piston end 189, applying a biasing force against the piston 134 that moves the piston 134 into the fluid chamber 168. The piston 134 can generate a pressure increase within the pressure chamber 153 as it moves into the fluid chamber 168. The pressure increase can, in turn, apply an opening force to the discharge check valve 165 that opens the discharge check valve 165 upon reaching the discharge check valve crack pressure, while simultaneously sealing closed the suction check valve 162. Initially, in a priming action, no hydraulic fluid will have yet entered into the pressure chamber 153 from the reservoir 150. In a second suction stroke, the biasing spring 137 acts upon the piston 134 and the trigger 131 to return both to the previously described neutral position. The reverse movement of the piston 134 out of the fluid chamber 168 generates a negative pressure force within the pressure chamber 153 that forces close the discharge check valve 165. If the crack pressure for the suction check valve 162 is reached, the reverse movement will also bias the suction check valve 162 to an open configuration, thereby pulling fluid from the reservoir 150 into the fluid chamber 168. A repeated pressure stroke biases the suction check valve 162 to a closed configuration, thereby preventing fluid from re-entering the reservoir 150 while simultaneously forcing the fluid contained in the fluid chamber 168 through the open discharge check valve 165 towards the outlet control valve body 156. As has been previously described, the fluid flow is able to be selectively controlled at the outlet control valve body 156 by the positioning of the outlet control switch 171 and can flow through one or more of the valve outlet channels 204 as desired. Repeated pressure and suction strokes maintain a pressurized fluid flow through the hydraulic injector 125 and out outlet ports 159 where the fluid can continue to flow through the tubing 52 into the cement cartridge assembly or assemblies as previously described thus providing a hydraulic force sufficient to move a quantity of bone cement from the cartridge assembly to the target anatomical location(s), for example, the vertebra of a spine in a bi-pedicular Vertebroplasty / Cementoplasty procedure.

Although the present disclosure has been described in accordance with several embodiments, it should be understood that various changes, substitutions, and alterations can be - made herein without departing from the scope of the present disclosure, for instance as indicated by the dependent claims. Thus, it should be appreciated that the scope of the present disclosure is not intended to be limited to the particular embodiments of the process, machine, manufacture, and composition of matter, methods and steps described herein. For instance, the various features as described above in accordance with one embodiment can be incorporated into the other embodiments unless indicated otherwise or contrary to the appended claims. Furthermore, as one of ordinary skill in the art will readily appreciate from the present disclosure, processes, machines, manufacture, composition of matter, methods, or steps, presently existing or later to be developed that perform substantially the same function or achieve substantially the same result as the corresponding embodiments described herein may be utilized according to the present disclosure.

It will be appreciated by those skilled in the art that various modifications and alterations of the invention can be made without departing from the scope of the appended claims. Some of these have been discussed above and others will be apparent to those skilled in the art.

## Claims

1. A hydraulic injection system (50) configured to inject bone cement, the hydraulic injection system comprising:
a cement cartridge assembly (59) including a plunger (99) and a cartridge body (63) that defines an interior cavity (96) that slidably supports the plunger (99) such that the plunger (99) defines a barrier surface that is movable in the interior cavity (96) from a first position to a second position so as to eject fluid out the interior cavity (96);
a hydraulic injector (125) comprising a housing (128) that defines a fluid chamber (168), a fluid reservoir (150) in fluid communication with the fluid chamber (168), a piston (134) that has a first piston end (186) that is at least partially disposed within the fluid chamber (168), a suction check valve (162) that is disposed between the fluid reservoir (150) and the fluid chamber (168), wherein the suction check valve (162) allows fluid to travel along a direction from the fluid reservoir (150) toward the fluid chamber (168), and prevents fluid from traveling from the fluid chamber (168) to the fluid reservoir (150), and at least one outlet port (159) that is in fluid tight communication with the fluid chamber (168); and
at least one conduit (52) configured to fluidly interconnect the at least one outlet port (159) of the hydraulic injector (125) and the cement cartridge assembly (59) so as to place the fluid chamber (168) and the interior cavity (96) of the cartridge body (63) in fluid communication with each other,
**characterized in that** the cement cartridge assembly (59) includes at least two cement cartridges (60).

2. The hydraulic system according to claim 1, wherein the cement cartridge assembly (59) comprises a cartridge cap (66) disposed in fluid-tight communication with the cartridge body (63), and the interior cavity (96) having a discharge opening (105) located at a distal end (93) of the cartridge body (63).

3. The hydraulic system according to claim 1 or 2, wherein a distal end (93) of the cartridge body (63) comprises a needle fastener (108) that is configured to connect to a cannulated needle (110) so as to place the cannulated needle (110) in fluid communication with the interior cavity (96).

4. The hydraulic injection system of any one of claims 1 to 3, wherein the suction check valve (162) is a low crack pressure check valve preferably having a cracking pressure ranging between about 0 kPa and 100 kPa.

5. The hydraulic injection system of any one of claims 1 to 4, wherein the hydraulic injector (125) further includes an outlet control valve assembly (155) disposed between the fluid chamber (168) and the at least one outlet port (159), the outlet control valve assembly (155) including an outlet control valve body (156) and an outlet control switch (171), the outlet control switch (171) including a plug portion (198), wherein the plug portion (198) of the outlet control switch (171) is seated in the outlet control valve body (156), and the outlet control valve body (156) defining a valve inlet channel (201) and at least one valve outlet channel (204), and wherein the plug portion (198) includes at least two switch channels (207) configured to selectively align with the valve inlet channel (201) and the at least one valve outlet channel (204) in the outlet control valve body (156) such that a fluid flow between the fluid chamber (168) and the at least one outlet port (159) can be controlled by a selective alignment of the outlet control switch (171).

6. The hydraulic injection system of claim 5, wherein the at least one valve outlet channel (204) and the at least one outlet port (159) includes two outlet valve channels and two outlet ports, respectively.

7. The hydraulic injection system of any one of claims 1 to 6, wherein the hydraulic injection system (50) is configured to generate a pressure in a range of about 0 to at least 200 bars, preferably in a range of about 0 to at least 100 bars, more preferably in a range of about 0 to at least 80 bars, more preferably in a range of about 0 to at least 50 bars, still more preferably in a range of about 0 to at least 20 bars.

8. The hydraulic injection system of any one of claims 1 to 4, wherein the hydraulic injector (125) further includes a discharge check valve (165) and wherein the discharge check valve (165) is disposed between the fluid chamber (168) and the at least one outlet port (159) along a fluid flowpath such that the fluid chamber (168) and the at least one outlet port (159) are in fluid tight communication with one another.

9. The hydraulic injection system of claims 5 or 6, wherein the hydraulic injector (125) further includes a discharge check valve (165) and wherein the discharge check valve (165) is disposed between the fluid chamber (168) and the outlet control valve assembly (155) along a fluid flowpath such that the fluid chamber (168) and the outlet control valve assembly (155) are in fluid tight communication with one another.

10. The hydraulic injection system of any one of claims 1 to 9, wherein the at least one conduit (52) has a length that is at least 50 cm.

11. The hydraulic injection system of any one of claims 1 to 10, wherein the barrier surface is disposed between a first fluid portion (113) of the interior cavity (96) located proximally to the plunger (99) and a second cement portion (116) of the cavity (96) located distal to the plunger (99), wherein the second cement portion (116) can house a quantity of bone cement to be injected into a target anatomical location.

12. The hydraulic injection system of any one of claims 1 to 11, further comprising a cannulated needle (110) configured to be coupled to the cement cartridge assembly (59).

13. The hydraulic injection system of any one of claims 1 to 12, with claim 2, further comprising a hydraulic fluid conduit (53) at least partially disposed within the cartridge cap (66), wherein the hydraulic fluid conduit is configured to fluidly interconnect the at least one conduit (52) and the interior cavity (96).

14. The hydraulic injection system of any one of claims 1 to 13, wherein the piston (134) is configured to move with respect to the housing (128) between a first position and a second position such that movement of the piston (134) from the first position to the second position causes hydraulic fluid in the fluid chamber (168) to be ejected out of the housing (128) through the at least one outlet port (159), and movement of the piston (134) from the second position to the first position causes hydraulic fluid in the fluid reservoir (150) to travel to the fluid chamber (168) through the suction check valve (162).

15. The hydraulic injection system of any one of claims 1 to 14, with claim 2, wherein the cartridge cap (66) defines an inner channel (81) and a transverse channel (31) that is in fluid communication with the inner channel (81), the transverse channel (31) leads to the outside environment, and the cement cartridge assembly (59) includes a venting valve (84) that is configured to move between an open position in which the transverse channel (31) is blocked, thereby precluding gases in the inner channel (81) from exiting to the outside environment, and a closed position in which the transverse channel (31) is in fluid communication with the outside environment, thereby allowing gases in the inner channel (81) to escape to the outside environment.

## Patentansprüche

1. Hydraulisches Injektionssystem (50), das eingerichtet ist, Knochenzement zu injizieren, wobei das hydraulische Injektionssystem umfasst:
eine Zementkartuschenanordnung (59), enthaltend einen Stempel (99) und einen Kartuschenkörper (63), der eine innere Kavität (96) definiert, die den Stempel (99) verschieblich trägt, so dass der Stempel (99) eine Grenzoberfläche definiert, die in der inneren Kavität (96) von einer ersten Position in eine zweite Position beweglich ist, um Fluid aus der inneren Kavität (96) auszugeben,
einen hydraulischen Injektor (125), umfassend ein Gehäuse (128), das eine Fluidkammer (168) definiert, ein Fluidreservoir (150) in Fluidkommunikation mit der Fluidkammer (168), einen Kolben (134), der ein erstes Kolbenende (186) aufweist, das zumindest teilweise in der Fluidkammer (168) angeordnet ist, ein Saugrückschlagventil (162), das zwischen dem Fluidreservoir (150) und der Fluidkammer (168) angeordnet ist, wobei es das Saugrückschlagventil (162) erlaubt, dass Fluid entlang einer Richtung von dem Fluidreservoir (150) zu der Fluidkammer (168) läuft, und verhindert, dass Fluid von der Fluidkammer (168) zu dem Fluidreservoir (150) läuft, und zumindest einen Auslassanschluss (159), der in fluiddichter Kommunikation mit der Fluidkammer (168) steht, und
zumindest eine Leitung (52), die eingerichtet ist, den zumindest einen Auslassanschluss (159) des hydraulischen Injektors (125) und die Zementkartuschenanordnung (59) miteinander zu verbinden, um die Fluidkammer (168) und die innere Kavität (96) des Kartuschenkörpers (63) in Fluidkommunikation miteinander zu bringen,
**dadurch gekennzeichnet, dass** die Zementkartuschenanordnung (59) zumindest zwei Zementkartuschen (60) enthält.

2. Hydraulisches System nach Anspruch 1, wobei die Zementkartuschenanordnung (59) eine Kartuschenkappe (66) umfasst, die in fluiddichter Kommunikation mit dem Kartuschenkörper (63) angeordnet ist, und die innere Kavität (96) eine Ausstoßöffnung (105) aufweist, die sich an einem distalen Ende (93) des Kartuschenkörpers (63) befindet.

3. Hydraulisches System nach Anspruch 1 oder 2, wobei ein distales Ende (93) des Kartuschenkörpers (63) ein Nadelbefestigungsmittel (108) umfasst, das eingerichtet ist, mit einer kanülierten Nadel (110) verbunden zu werden, um die kanülierte Nadel (110) in Fluidkommunikation mit der inneren Kavität (96) zu bringen.

4. Hydraulisches Injektionssystem nach einem der Ansprüche 1 bis 3, wobei das Saugrückschlagventil (162) ein Rückschlagventil mit niedrigem Öffnungsdruck ist, das vorzugsweise einen Öffnungsdruckbereich zwischen ungefähr 0 kPa und 100 kPa aufweist.

5. Hydraulisches Injektionssystem nach einem der Ansprüche 1 bis 4, wobei der hydraulische Injektor (125) des Weiteren eine Auslasssteuerungsventilanordnung (155) enthält, die zwischen der Fluidkammer (168) und dem zumindest einen Auslassanschuss (159) angeordnet ist, wobei die Auslasssteuerungsventilanordnung (155) einen Auslasssteuerungsventilkörper (156) und einen Auslasssteuerungsumschalter (171) enthält, wobei der Auslasssteuerungsumschalter (171) einen Steckerteil (198) enthält, wobei der Steckerteil (198) des Auslasssteuerungsumschalters (171) in dem Auslasssteuerungsventilkörper (156) sitzt, und der Auslasssteuerungsventilkörper (156) einen Ventileinlasskanal (201) und zumindest einen Ventilauslasskanal (204) definiert, und wobei der Steckerteil (198) zumindest zwei Umschaltkanäle (207) enthält, die eingerichtet sind, wahlweise zu dem Ventileinlasskanal (201) und dem zumindest einen Ventilauslasskanal (204) in dem Auslasssteuerungsventilkörper (156) ausgerichtet zu werden, so dass ein Fluidfluss zwischen der Fluidkammer (168) und dem zumindest einen Auslassanschluss (159) durch eine wahlweise Ausrichtung des Auslasssteuerungsumschalters (171) gesteuert werden kann.

6. Hydraulisches Injektionssystem nach Anspruch 5, wobei der zumindest eine Ventilauslasskanal (204) und der zumindest eine Auslassanschluss (159) zwei Ventilauslasskanäle beziehungsweise zwei Auslassanschlüsse aufweisen.

7. Hydraulisches Injektionssystem nach einem der Ansprüche 1 bis 6, wobei das hydraulische Injektionssystem (50) eingerichtet ist, einen Druck in einem Bereich von ungefähr 0 bis zumindest 200 bar, vorzugsweise in einem Bereich von ungefähr 0 bis zumindest 100 bar, weiter vorzugsweise in einem Bereich von ungefähr 0 bis zumindest 80 bar, weiter vorzugsweise in einem Bereich von ungefähr 0 bis zumindest 50 bar, noch weiter vorzugsweise in einem Bereich von ungefähr 0 bis zumindest 20 bar zu erzeugen.

8. Hydraulisches Injektionssystem nach einem der Ansprüche 1 bis 4, wobei der hydraulische Injektor (125) des Weiteren ein Ausstoßrückschlagventil (165) enthält und wobei das Ausstoßrückschlagventil (165) zwischen der Fluidkammer (168) und dem zumindest einen Auslassanschluss (159) entlang eines Fluidflusspfades angeordnet ist, so dass die Fluidkammer (168) und der zumindest eine Auslassanschluss (159) in fluiddichter Kommunikation miteinander sind.

9. Hydraulisches Injektionssystem nach Anspruch 5 oder 6, wobei der hydraulische Injektor (125) des Weiteren ein Ausstoßrückschlagventil (165) enthält und wobei das Ausstoßrückschlagventil (165) zwischen der Fluidkammer (168) und der Auslasssteuerungsventilanordnung (155) entlang eines Fluidflusspfades angeordnet ist, so dass die Fluidkammer (168) und die Auslasssteuerungsventilanordnung (155) in fluiddichter Kommunikation miteinander sind.

10. Hydraulisches Injektionssystem nach einem der Ansprüche 1 bis 9, wobei die zumindest eine Leitung (52) eine Länge aufweist, die zumindest 50 cm beträgt.

11. Hydraulisches Injektionssystem nach einem der Ansprüche 1 bis 10, wobei die Grenzoberfläche zwischen einem ersten Fluidteil (113) der inneren Kavität (96), der sich proximal zu dem Stempel (99) befindet, und einem zweiten Zementteil (116) der Kavität (96), der sich distal zu dem Stempel (99) befindet, angeordnet ist, wobei der zweite Zementteil (116) eine Menge von Knochenzement beherbergen kann, die in eine anatomische Zielregion injiziert werden soll.

12. Hydraulisches Injektionssystem nach einem der Ansprüche 1 bis 11, des Weiteren umfassend eine kanülierte Nadel (110), die eingerichtet ist, an die Zementkartuschenanordnung (59) gekoppelt zu werden.

13. Hydraulisches Injektionssystem nach einem der Ansprüche 1 bis 12, mit Anspruch 2, des Weiteren umfassend eine hydraulische Fluidleitung (53), die zumindest teilweise in der Kartuschenkappe (66) angeordnet ist, wobei die hydraulische Fluidleitung eingerichtet ist, die zumindest eine Leitung (52) und die innere Kavität (96) fluidleitend zu verbinden.

14. Hydraulisches Injektionssystem nach einem der Ansprüche 1 bis 13, wobei der Kolben (134) eingerichtet ist, sich bezüglich des Gehäuses (128) zwischen einer ersten Position und einer zweiten Position zu bewegen, so dass eine Bewegung des Kolbens (134) von der ersten Position in die zweite Position bewirkt, das hydraulisches Fluid in der Fluidkammer (168) aus dem Gehäuse (128) durch den zumindest einen Auslassanschluss (159) ausgegeben wird, und eine Bewegung des Kolbens (134) von der zweiten Position in die erste Position bewirkt, dass hydraulisches Fluid in dem Fluidreservoir (150) durch das Saugrückschlagventil (162) zu der Fluidkammer (168) läuft.

15. Hydraulisches Injektionssystem nach einem der Ansprüche 1 bis 14, mit Anspruch 2, wobei die Kartuschenkappe (66) einen inneren Kanal (81) und einen querlaufenden Kanal (31), der in Fluidkommunikation mit dem inneren Kanal (81) ist, definiert, wobei der querlaufende Kanal (31) in die äußere Umgebung führt, und die Zementkartuschenanordnung (59) ein Belüftungsventil (84) enthält, das eingerichtet ist, sich zwischen einer offenen Position, in welcher der querlaufende Kanal (31) blockiert ist, wodurch verhindert wird, dass Gase in dem inneren Kanal (81) in die äußere Umgebung austreten, und einer geschlossenen Position, in welcher der querlaufende Kanal (31) in Fluidkommunikation mit der äußeren Umgebung ist, wodurch erlaubt wird, dass Gase in dem inneren Kanal (81) in die äußere Umgebung entweichen, zu bewegen.

## Revendications

1. Système d'injection hydraulique (50) configuré pour injecter du ciment osseux, le système d'injection hydraulique comprenant :
un ensemble de cartouche de ciment (59) comprenant un piston plongeur (99) et un corps de cartouche (63) qui définit une cavité intérieure (96) qui supporte de manière coulissante le piston plongeur (99) de sorte que le piston plongeur (99) définit une surface de barrière qui est mobile dans la cavité intérieure (96) d'une première position à une seconde position afin d'éjecter du fluide hors de la cavité intérieure (96) ;
un injecteur hydraulique (125) comprenant un boîtier (128) qui définit une chambre de fluide (168), un réservoir de fluide (150) en communication de fluide avec la chambre de fluide (168), un piston (134) qui a une première extrémité de piston (186) qui est au moins partiellement disposée à l'intérieur de la chambre de fluide (168), une soupape d'aspiration anti-retour (162) qui est disposée entre le réservoir de fluide (150) et la chambre de fluide (168), dans lequel ladite valve d'aspiration anti-retour (162) permet au fluide de se déplacer le long d'une direction allant du réservoir de fluide (150) vers la chambre de fluide (168), et empêche le fluide de se déplacer de la chambre de fluide (168) au réservoir de fluide (150), et au moins un orifice de sortie (159) qui est en communication étanche au fluide avec la chambre de fluide (168) ; et
au moins un conduit (52) configuré pour interconnecter de manière fluide le au moins un orifice de sortie (159) de l'injecteur hydraulique (125) et l'ensemble de cartouche de ciment (59) afin de placer la chambre de fluide (168) et la cavité intérieure (96) du corps de cartouche (63) en communication de fluide entre elles,
**caractérisé en ce que** l'ensemble de cartouche de ciment (59) comprend au moins deux cartouches de ciment (60).

2. Système hydraulique selon la revendication 1, dans lequel l'ensemble de cartouche de ciment (59) comprend un capuchon de cartouche (66) disposé en communication étanche au fluide avec le corps de cartouche (63), et la cavité intérieure (96) ayant une ouverture de décharge (105) positionnée à une extrémité distale (93) du corps de cartouche (63).

3. Système hydraulique selon la revendication 1 ou 2, dans lequel une extrémité distale (93) du corps de cartouche (63) comprend une fixation d'aiguille (108) qui est configurée pour se raccorder à une aiguille à canule (110) afin de placer l'aiguille à canule (110) en communication de fluide avec la cavité intérieure (96).

4. Système d'injection hydraulique selon l'une quelconque des revendications 1 à 3, dans lequel la valve d'aspiration anti-retour (162) est une valve anti-retour à faible pression d'ouverture ayant de préférence une pression d'ouverture comprise entre environ 0 kPa et 100 kPa.

5. Système d'injection hydraulique selon l'une quelconque des revendications 1 à 4, dans lequel l'injecteur hydraulique (125) comprend en outre un ensemble de valve de commande de sortie (155) disposé entre la chambre de fluide (168) et le au moins un orifice de sortie (159), l'ensemble de valve de commande de sortie (155) comprenant un corps de valve de commande de sortie (156) et un commutateur de commande de sortie (171), le commutateur de commande de sortie (171) comprenant une partie de bouchon (198), dans lequel la partie de bouchon (198) du commutateur de commande de sortie (171) est installée dans le corps de valve de commande de sortie (156) et le corps de valve de commande de sortie (156) définissant un canal d'entrée de valve (201) et au moins un canal de sortie de valve (204), et dans lequel la partie de bouchon (198) comprend au moins deux canaux de commutateur (207) configurés pour s'aligner sélectivement avec le canal d'entrée de valve (201) et le au moins un canal de sortie de valve (204) dans le corps de valve de commande de sortie (156) de sorte qu'un écoulement de fluide entre la chambre de fluide (168) et le au moins un orifice de sortie (159) peut être régulé par un alignement sélectif du commutateur de commande de sortie (171).

6. Système d'injection hydraulique selon la revendication 5, dans lequel le au moins un canal de sortie de valve (204) et le au moins un orifice de sortie (159) comprend deux canaux de valve de sortie et deux orifices de sortie, respectivement.

7. Système d'injection hydraulique selon l'une quelconque des revendications 1 à 6, dans lequel le système d'injection hydraulique (50) est configuré pour générer une pression dans une plage d'environ 0 à environ 200 bar, de préférence dans une plage d'environ 0 à au moins 100 bar, encore de préférence dans une plage d'environ 0 à au moins 80 bar, encore de préférence dans une plage d'environ 0 à au moins 50 bar, encore de préférence dans une plage d'environ 0 à au moins 20 bar.

8. Système d'injection hydraulique selon l'une quelconque des revendications 1 à 4, dans lequel l'injecteur hydraulique (125) comprend en outre une valve anti-retour de décharge (165) et dans lequel la valve anti-retour de décharge (165) est disposée entre la chambre de fluide (168) et le au moins un orifice de sortie (159) le long d'une trajectoire d'écoulement de fluide de sorte que la chambre de fluide (168) et le au moins un orifice de sortie (159) sont en communication étanche au fluide entre eux.

9. Système d'injection hydraulique selon les revendications 5 ou 6, dans lequel l'injecteur hydraulique (125) comprend en outre une valve anti-retour de décharge (165) et dans lequel la valve anti-retour de décharge (165) est disposée entre la chambre de fluide (168) et l'ensemble de valve de commande de sortie (155) le long d'une trajectoire d'écoulement de fluide de sorte que la chambre de fluide (168) et l'ensemble de valve de commande de sortie (155) sont en communication étanche au fluide entre eux.

10. Système d'injection hydraulique selon l'une quelconque des revendications 1 à 9, dans lequel le au moins un conduit (52) a une longueur qui est d'au moins 50 cm.

11. Système d'injection hydraulique selon l'une quelconque des revendications 1 à 10, dans lequel la surface de barrière est disposée entre une première partie de fluide (113) de la cavité intérieure (96) positionnée à proximité du piston plongeur (99) et une seconde partie de ciment (116) de la cavité (96) positionnée à distance du piston plongeur (99), dans lequel la seconde partie de ciment (116) peut loger une quantité de ciment osseux à injecter dans un emplacement anatomique cible.

12. Système d'injection hydraulique selon l'une quelconque des revendications 1 à 11, comprenant en outre une aiguille à canule (110) configurée pour être couplée à l'ensemble de cartouche de ciment (59).

13. Système d'injection hydraulique selon l'une quelconque des revendications 1 à 12, avec la revendication 2, comprenant en outre un conduit de fluide hydraulique (53) disposé au moins partiellement à l'intérieur du capuchon de cartouche (66), dans lequel le conduit de fluide hydraulique est configuré pour interconnecter de manière fluide le au moins un conduit (52) et la cavité intérieure (96).

14. Système d'injection hydraulique selon l'une quelconque des revendications 1 à 13, dans lequel le piston (134) est configuré pour se déplacer par rapport au boîtier (128) entre une première position et une seconde position de sorte que le mouvement du piston (134) de la première position à la seconde position amène le fluide hydraulique dans la chambre de fluide (168) à être éjecté du boîtier (128) par le au moins un orifice de sortie (159) et le mouvement du piston (134) de la seconde position à la première position amène le fluide hydraulique dans le réservoir de fluide (150) à se déplacer dans la chambre de fluide (168) à travers la valve anti-retour d'aspiration (162).

15. Système d'injection hydraulique selon l'une quelconque des revendications 1 à 14, avec la revendication 2, dans lequel le capuchon de cartouche (66) définit un canal interne (81) et un canal transversal (31) qui est en communication de fluide avec le canal interne (81), le canal transversal (31) mène à un environnement extérieur, et l'ensemble de cartouche de ciment (59) comprend une valve d'aération (84) qui est configurée pour se déplacer entre une position ouverte dans laquelle la canal transversal (31) est bloqué, empêchant ainsi les gaz dans le canal interne (81) de sortir dans l'environnement extérieur, et une position fermée dans laquelle le canal transversal (31) est en communication de fluide avec l'environnement extérieur, permettant ainsi aux gaz dans le canal interne (81) de s'échapper dans l'environnement extérieur.
